(19) Europäisches Patentamt
European Patent Office
Office européen des brevets

(11) Publication number: **0 509 845 A1**

# (12) EUROPEAN PATENT APPLICATION

(21) Application number: **92303505.9**

(22) Date of filing: **21.04.92**

(51) Int. Cl.$^5$: **C07D 413/04**, C07D 417/04, A61K 31/50

(30) Priority: **18.04.91 US 687353**

(43) Date of publication of application: **21.10.92 Bulletin 92/43**

(84) Designated Contracting States: **AT BE CH DE DK ES FR GB GR IT LI NL PT**

(71) Applicant: **ORTHO PHARMACEUTICAL CORPORATION**
**U.S. Route 202 P.O. Box 300**
**Raritan New Jersey 08869-0602 (US)**

(72) Inventor: **Combs, Donald W.**
**43 Seymour Terrace**
**Piscataway, New Jersey 08854 (US)**

(74) Representative: **Fisher, Adrian John et al**
**CARPMAELS & RANSFORD 43 Bloomsbury Square**
**London WC1A 2RA (GB)**

(54) **(+) and (-) enantiomers of 5-aliphatic-6-(benzoxazinyl- or benzothiazinyl)-2,3,4,5-tetrahydropyridazin-3-ones.**

(57) The present invention relates to (+) and (-) enantiomers of compounds of the formula :

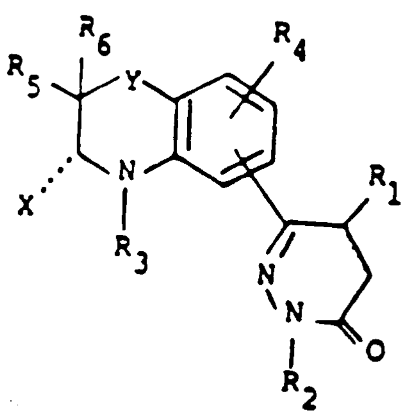

I

as further defined herein. The compounds are useful as cardiotonic and vasodilating agents and as inhibitors of phosphodiesterase fraction III and platelet aggregation. In addition, the compounds are active as smooth muscle relaxants and bronchodilators.

EP 0 509 845 A1

## CROSS REFERENCE

United States patent application No. 63l,550 filed December 2l, l990 is cross-referenced to the instant application.

## BACKGROUND OF THE INVENTION

## FIELD OF THE INVENTION

The present invention relates to (+) and (-) enantiomers of compounds of the formula:

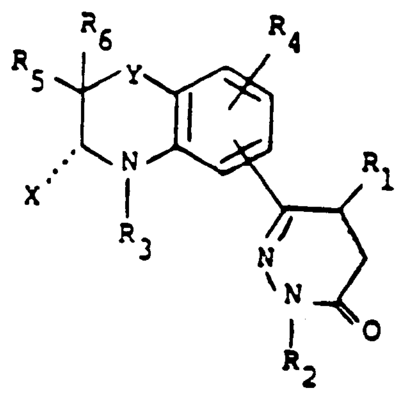

I

as further defined herein. The compounds are useful as cardiotonic and vasodilating agents and as inhibitors of phosphodiesterase fraction III and platelet aggregation. In addition, the compounds are active as smooth muscle relaxants and bronchodilators.

## DESCRIPTION OF THE PRIOR ART

Quinoline substituted pyridazin-3-ones have been shown to be cardiotonic agents and platelet aggregation inhibitors. Published European Patent Application No. l55,798 and British Patent No. 2,03l,404 describe compounds of the formula:

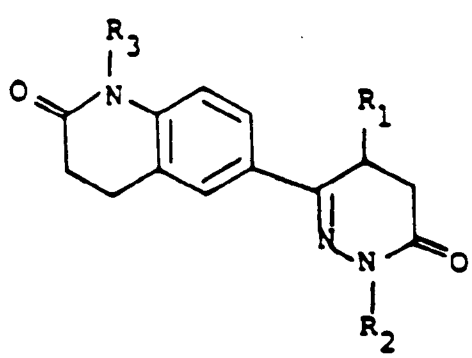

where $R_l$, $R_2$ and $R_3$ may be H or lower alkyl.

U. S. patent 4,562,l90 describes benzothiazole substituted pyridazin-3-ones of the formula

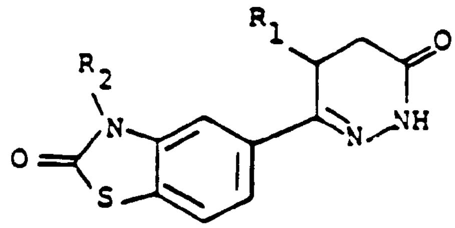

III

where $R_l$ is $C_l$-$C_6$ alkyl and $R_2$ is H, $C_l$-$C_6$ alkyl or aryl.

## SUMMARY OF THE INVENTION

The present invention is directed to (+) and (-) enantiomers of 5-aliphatic-6-(benzoxazinyl- and benzothiazinyl)-2,3,4,5-tetrahydropyridazin-3-ones of the general formula:

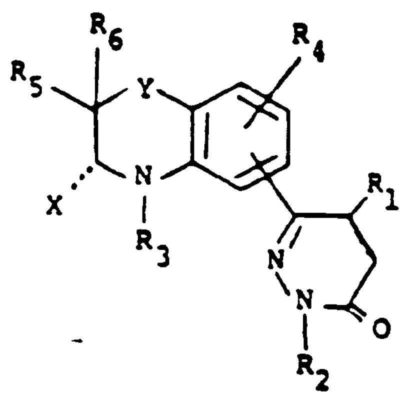

I

wherein

X is $H_2$ or O;

Y is O or S;

$R_l$ is $C_{l-6}$ straight- or branched-chain alkyl or $C_{3-6}$ cycloalkyl;

$R_2$ is $C_{l-6}$ straight- or branched-chain alkyl, $C_{3-6}$ cycloalkyl or $C_{3-6}$ alkenyl;

$R_3$ is H, $C_{l-6}$ straight- or branched-chain alkyl or $C_{3-6}$ cycloalkyl, and when X is $H_2$, $R_3$ may also be $C_2$-$C_6$ acyl, arylacyl such as benzoyl and phenylacetyl, or alkanesulfonyl such as methanesulfonyl;

$R_4$ is H, halogen, $C_{l-6}$ straight- or branched-chain alkyl, $C_{3-6}$ cycloalkyl or $C_{l-6}$ alkoxy;

$R_5$ and $R_5$ are each H, $C_{l-6}$ straight- or branched-chain alkyl or $C_{3-6}$ cycloalkyl.

The compounds of formula I are useful as cardiotonic agents having a long duration of activity and are very potent inhibitors of phosphodiesterase fraction III.

## DETAILED DESCRIPTION OF THE INVENTION

The invention in its broadest aspects relates to enantiomeric pyridazinone compounds which exhibit cardiotonic activity, vasodilating activity, platelet anti-aggregatory activity and phosphodiesterase fraction III inhibitory activity. The enantiomeric pyridazinone compounds of the invention demonstrating these activities are shown by formula I above.

The (+) and (-) signs denote that the enantiomeric orientation relative to an asymmetric carbon in the compound of formula I is respectively S and R.

The aliphatic group in the compound of the formula (I) is represented by $R_l$, i.e, $C_{l-6}$ straight- or branched-chain alkyl or $C_{3-6}$ cycloalkyl.

The preferred compounds of the present invention are those wherein $R_l$ is $CH_3$, $R_2$ and $R_3$ are hydrogen, $R_4$, $R_5$ and $R_6$ are H or $CH_3$, X is O, Y is O, the pyridazinone ring is attached at C-7 of the benzoxazine or the benzothiazine ring and the enantiomeric orientation relative to the carbon bearing $R_l$ is R.

The compounds of the invention are prepared by resolving a racemic mixture of the compounds utilizing chiral column chromatography procedures, e.g., using normal phase column chromatography conditions. Furthermore precursors of the final compounds can be resolved employing the aforesaid method, and the resolved precursors are then converted to the corresponding enantiomeric compound of the formula I.

When (+), (-) and racemic, (±), 6-(3,4-dihydro-3-oxo-l,4(2H)-benzoxazin-7-yl)-2,3,4,5-tetrahydro-5-methyl-pyridazin-3-one (bemoradan), which is a compound of the formula (I), were tested for their potency as inhibitors of canine cardiac cyclic nucleotide phosphodiesterase (PDE) fraction III and platelet aggregation in vitro as well as regarding in vivo inotropic effects it was discerned that the (-) isomer, is the more potent of the two enantiomers by approximately one order of magnitude (See, Table I). The (-) isomer is comparable in potency to the racemic bemoradan, i.e., in vivo as an inotropic agent and in vitro as an inhibitor of platelet aggregation and PDE fraction III. In sharp contrast, the (+) isomer is consistently less potent than the (-) isomer or the racemate. The difference in potency between the (+) isomer and the (-) isomer varies from 7x in vivo to 44x in PDE III inhibition. The (-) isomer is also more active at reducing the mean arterial blood pressure than the (+) isomer.

TABLE I

Summary Comparison of Bemoradan and its Enantiomers

| Test Procedure | (-) isomer | (racemate) | (+) isomer | Potency Difference (-vs+) |
|---|---|---|---|---|
| PDE III inhibition ($IC_{50}$) | 0.18 μM | 0.71 μM | 8.0 μM | 44x |
| Platelet aggregation ($IC_{50}$) | | | | |
| vs. ADP | 0.32 μM | 0.65 μM | 3.8 μM | 12x |
| vs. 5-HT (serotonin) | 0.50 μM | 0.40 μM | 8.0 μM | 16x |
| In vivo inotropic $ED_{40}$, i.v. | 5.7 μg/kg | 4.4 μg/kg | 42.3 μg/kg | 7x |

The starting materials for preparing the compounds of the present invention can be prepared as shown in Scheme I.

SCHEME 1

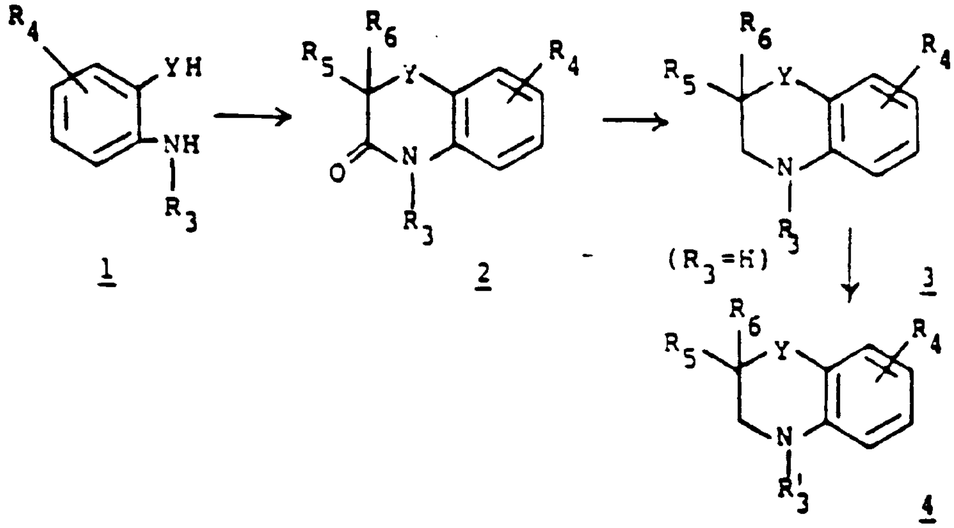


1 2 3 4

wherein $R'_3$ is R″CO or R″$SO_2$, wherein R″ is lower alkyl and the alkyl group contains I-6 carbon atoms, and $R_4$, $R_5$ and $R_5$ are as previously defined.

The benzoxazinone or benzothiazinone 2 is prepared from compound I by the procedure of Shridhar, Org. Prep. Proc. Int., I4, I95 (I982). Compound 2 is refluxed for several hours in one equivalent of diborane in tetrahydrofuran to produce the benzoxazine or benzothiazine 3. Compound 3, where $R_3$ is H, is treated with a sulfonyl or acyl compound such as methanesulfonyl chloride or acetyl chloride and pyridine in a solvent such as dichloromethane and refluxed for several hours to produce the benzoxazine or benzothiazine 4.

The racemic compounds of formula I can be prepared as shown in Schemes 2, 3 and 4.

SCHEME 2

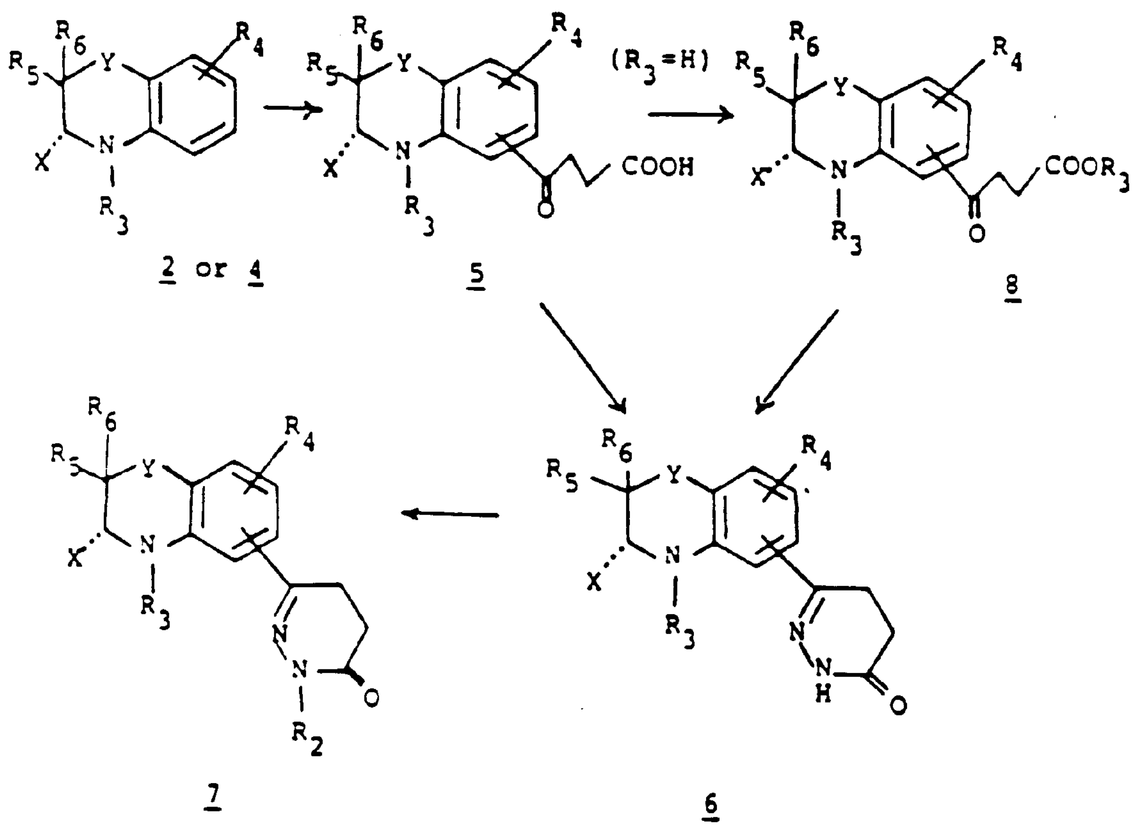


2 or 4

5

8

7

6

The benzoxazine or benzothiazine 2 or 4 is acylated by the method of Thyes, J. Med. Chem., 26, 800 (l983) using succinic anhydride to produce the compound 5. Compound 5 is refluxed for l-8 hours with 2.2 equivalents of hydrazine in an alcohol solvent such as methanol to give compound 6. Alternatively, compound 6 can be prepared by first esterifying compound 5 in alcoholic HCl to form compound 8 and then reacting compound 8 with hydrazine. Compound 6 can be alkylated at the 2-position of the pyridazinone ring by treatment in an inert solvent such as dimethylformamide with an alkali metal base such as sodium hydride and subsequent treatment with an alkyl halide, $R_2X$ wherein $R_2$ is as defined above and X is chloro, bromo or iodo, at about 0-40°C for about 0.5-8 hours to give compound 7. Alternatively, compound 5 (when $R_3$ is H) can be alkylated at the 4-position as described above to give compound 8. Compound 8 is refluxed with hydrazine to produce compound 6. The N-acylated derivative 7 (X = $H_2$; $R_3$ = acyl or sulfonyl) was prepared from 6 (X = $H_2$; $R_3$ = H) by treatment with a base such as triethylamine, and the appropriate acid chloride, such as acetyl chloride, methanesulfonyl chloride, benzoyl chloride, for example, as described above.

SCHEME 3

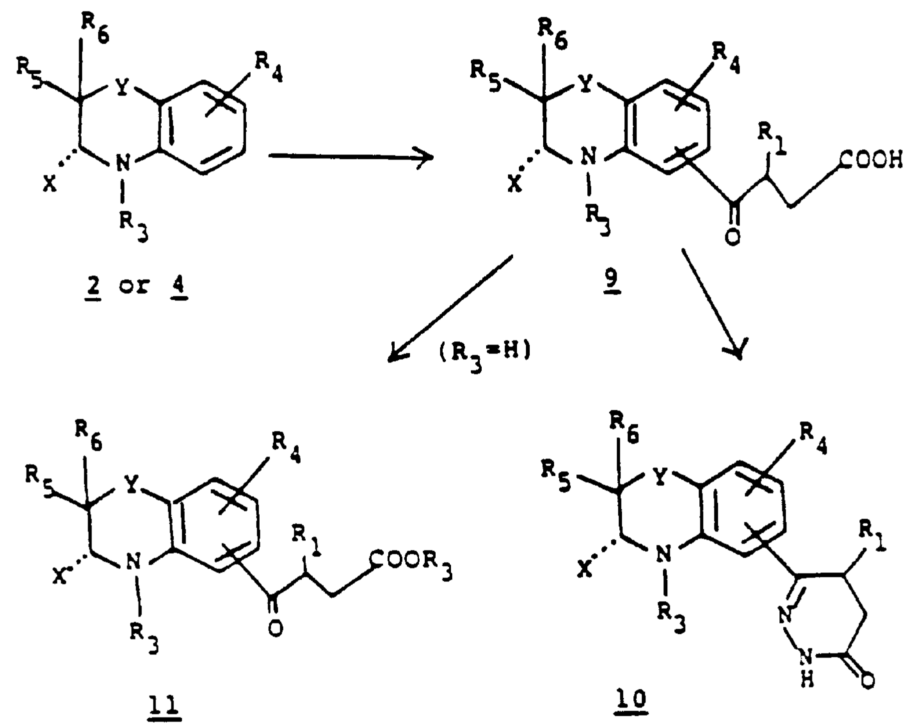


2 or 4

9

11

10

To prepare a 5-alkylated pyridazinone, the benzoxazine or benzothiazine 2 or 4 is acylated with propionyl chloride by the method of Thyes, supra, and the resulting product is converted to compound 9 by the method of McEvoy and Allen, J. Org. Chem., 38, 4044 (I973). Compound 9 is reacted with hydrazine or alkylated as described above to produce compounds I0 and II, respectively. Compound II can be reacted with hydrazine to give compound I0. Compound I0 can be alkylated at the 2-position of the pyridazinone ring or acylated at the 4-position of the benzoxazine or benzothiazine ring as described previously.

SCHEME 4

2 or 4

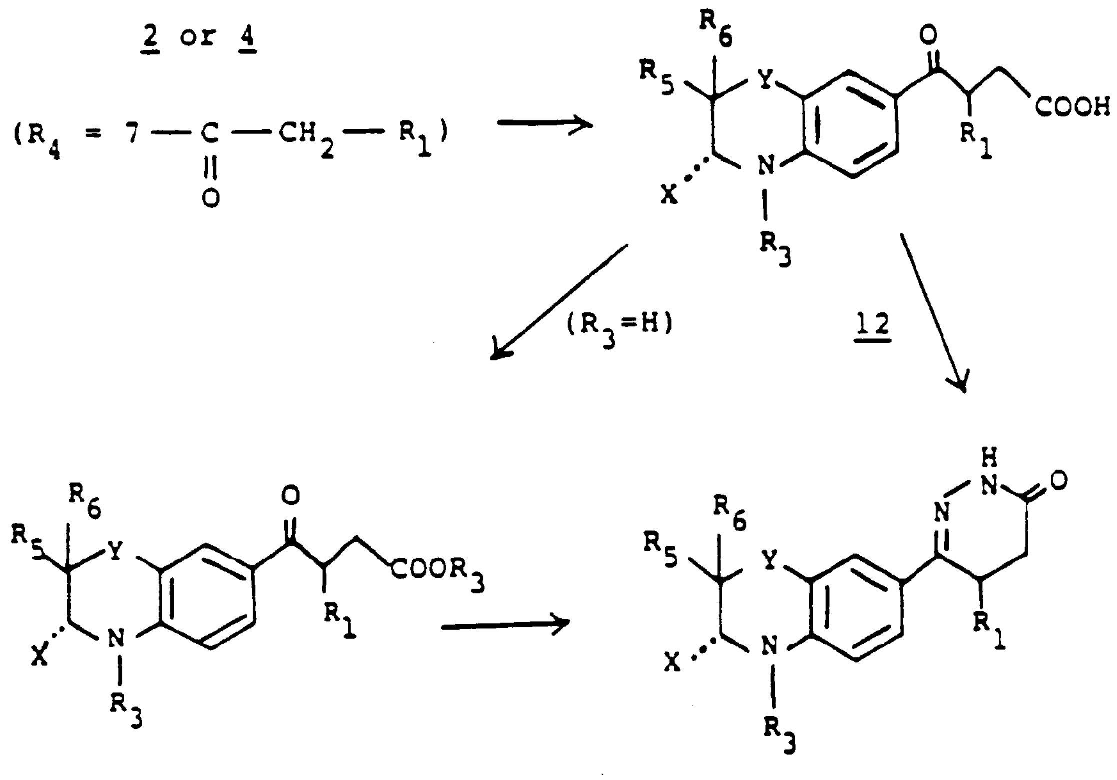


12

14

13

The benzoxazine or benzothiazine 2 or 4, when $R_4$ is -C(O)-$CH_2$-$R_1$ at the 7-position of the ring, is converted to compound 12 by the method of McEvoy and Allen, supra. Compound 12 is reacted with hydrazine or alkylated as described above to produce compounds 13 and 14, respectively. Compound 14 can be reacted with hydrazine to give compound 13. Compound 13 can be alkylated at the 2-position of the pyridazinone ring or acylated at the 4-position of the benzoxazine or benzothiazine ring as previously described.

Additional procedures for preparing racemic 6-(dihydro-3-oxo-1,4(2H)-benzoxazin-7-yl)-2,3,4,5-tetrahydropyridazin-3-ones are described in United States Patent Application No. 631,550 filed December 21, 1990 and that application is incorporated by reference herein.

Pharmaceutical compositions containing a compound of the present invention as the active ingredient in intimate admixture with a pharmaceutical carrier can be prepared according to conventional pharmaceutical compounding techniques. The carrier may take a wide variety of forms depending on the form of preparation desired for administration, e.g., intravenous, oral or parenteral. The composition may also be administered by means of an aerosol. In preparing the compositions in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. For parenterals, the carriers will usually comprise sterile water, though other ingredients, for example, to aid solubility or for preservative purposes, may be included, injectable suspensions may also be prepared, in which case appropriate liquid carriers, suspending agents and the like may be employed. The pharmaceutical compositions will generally contain dosage unit, e.g., tablet, capsule, powder, injection, teaspoonful and the like, from about 0.001 to about 10 mg/kg, and preferably from about 0.01 to about 0.1 mg/kg of the active ingredient.

The following examples describe the invention in greater particularity and are intended to be a way of illustrating but not limiting the invention.

EXAMPLE 1

3,4-Dihydro-7-(l-oxopropyl)-3-oxo-l,4(2H)-benzoxazine

4-amino-3-hydroxypropiophenone (32 g) was dissolved in 250 ml of methyl isobutyl ketone and 250 ml of water containing 40 g of sodium bicarbonate. Chloroacetyl chloride (l7 ml) was added to the rapidly stirring mixture at 0°C. The mixture was then heated at reflux for four hours. Upon cooling, the title compound was isolated by filtration and washed with ether. Yield: 35 g (88%), mp l74.5-l76°C.

The following compounds were prepared by the above procedure, using the appropriate starting materials:
3,4-dihyro-3-oxo-l,4(2H)-benzoxazine, mp l70-l7l°C;
3,4-dihydro-6-methyl-3-oxo-l,4(2H)-benzoxazine, mp 204.5-205.5°C;
3,4-dihydro-7-methyl-3-oxo-l,4(2H)-benzoxazine, mp l93-l95°C
3,4-dihydro-2-methyl-3-oxo-l,4(2H)-benzoxazine, mp l43-l45°C
3,4-dihydro-2,2-dimethyl-3-oxo-l,4(2H)-benzoxazine, mp l6l-l63°C;
3,4-dihydro-2,7-dimethyl-3-oxo-l,4(2H)-benzoxazine, mp l52-l53°C;
3,4-dihydro-4-(l-methylethyl)-3-oxo-l,4(2H)-benzoxazine, oil;
3,4-dihydro-4-cyclopentyl-3-oxo-l,4(2H)-benzoxazine, oil;
3,4-dihydro-2-methyl-4-(l-methylethyl)-3-oxo-l,4(2H)-benzoxazine, oil;
3,4-dihydro-2-methyl-4-cyclopentyl-3-oxo-l,4(2H)-benzoxazine, oil;
3,4-dihydro-7-(l-oxoethyl)-3-oxo-l,4(2H)-benzoxazine, mp l93-l96°C;
3,4-dihydro-3-oxo-l,4[2H]-benzothiazine;
3,4-dihydro-3-oxo-7-(l-oxopropyl)-l,4[2H]-benzothiazine;
3,4-dihydro-2-methyl-3-oxo-l,4[2H]-benzothiazine; and
3,4-didhyro-,2-dimethyl-3-oxo-l,4[2H]benzothiazine.

EXAMPLE 2

3,4-Dihydro-l,4(2H)-benzoxazine

3,4-Dihydro-3-oxo-l,4(2H)-benzoxazine was refluxed for several hours in one equivalent of diborane in tetrahydrofuran. Excess sodium hydroxide solution was added, the product was extracted with ether and the solvent was evaporated to give the title compound as an oil.

The following compounds were prepared by the above procedure, using the appropriate starting materials:
3,4-dihydro-6-methyl-l,4(2H)-benzoxazine and
3,4-dihydro-2-methyl-l,4(2H)-benzoxazine.

EXAMPLE 3

3,4-Dihydro-2,7-dimethyl-4-(l-oxoethyl)-l,4(2H)-benzoxazine

3,4-Dihydro-2,7-dimethyl-l,4(2H)-benzoxazine was dissolved in dichloromethane, and one equivalent each of acetyl chloride and triethylamine was added in that order. The mixture was refluxed for several hours, cooled and washed with water, and then with saturated $NaHCO_3$ solution. Evaporation of the organic layer provided the product, mp 60.5-63°C.

The following compounds were prepared by the above procedure, using the appropriate starting materials:
3,4-dihydro-4-methanesulfonyl-l,4(2H)-benzoxazine, mp 74.5-77°C;
3,4-dihydro-4-(l-oxoethyl)-l,4(2H)-benzoxazine, oil; and
3,4-dihydro-2-methyl-4-(l-oxoethyl)-l,4(2H)-benzoxazine, mp 80-82°C.

EXAMPLE 4

4-Oxo-4-(3,4-dihydro-2-methyl-3-oxo-l,4(2H)-benzoxazine-6-yl)butyric acid

3,4-Dihydro-2-methyl-3-oxo-l,4(2H)-benzoxazine (ll.4 g) and succinic anhydride (7 g) were added to 93 g of aluminum chloride and l5.3 ml of dimethylformamide. The mixture was stirred at 70°C for 2.5 hours and then poured onto ice, giving a solid which was collected by filtration and washed with water. Drying under vacuum gave l6.5 g of the title compound (90% yield), mp l98-200°C.

The following compounds were prepared by the above procedure, using the appropriate starting materials:

4-oxo-4-(3,4-dihydro-3-oxo-l,4(2H)-benzoxazine-6-yl)butyric acid, mp 206-208°C;
4-oxo-4-(3,4-dihydro-2,2-dimethyl-3-oxo-l,4(2H)-benzoxazine-6-yl)butyric acid, mp 226-228°C;
4-oxo-4-(3,4-dihydro-2,7-dimethyl-3-oxo-l,4(2H)-benzoxazin-6-yl)butyric acid;
4-oxo-4-(3,4-dihydro-4-methanesulfonyl-l,4(2H)-benzoxazin-6-yl)butyric acid, mp l84-l87°C;
4-oxo-4-(3,4-dihydro-4-(l-oxoethyl)-l,4(2H)-benzoxazin-6-yl)butyric acid, mp l43-l44.5°C;
4-oxo-4-(3,4-dihydro-6-methyl-3-oxo-l,4(2H)-benzoxazin-8-yl)butyric acid; and
4-oxo-4-(3,4-dihydro-6-methyl-3-oxo-l,4(2H)-benzoxzain-7-yl)butyric acid.

EXAMPLE 5

Methyl 4-oxo-4-(3,4-dihydro-4-methyl-3-oxo-l,4(2H)-benzoxazine-6-yl)butyrate

3,4-Dihydro-3-oxo-l,4(2H)-benzoxazine was alkylated by dissolving the acid in dimethylformamide and adding two equivalents of 60% sodium hydride in oil suspension. After one-half hour, two equivalents of methyl iodide were added. The mixture was stirred under nitrogen for l2 hours, then poured into water. The product was collected by extraction into ethyl acetate and evaporation of the solvent, mp l39-l50°C.

The following compounds were prepared by the above procedure, using the appropriate starting materials:
methyl 4-oxo-4-(3,4-dihydro-2,4-dimethyl-3-oxo-l,4(2H)-benzoxazine-6-yl)butyrate, oil;
methyl 4-oxo-4-(3,4-dihydro-4,7-dimethyl-3-oxo-l,4(2H)-benzoxazin-6-yl)butyrate, oil;
methyl 4-oxo-4-(3,4-dihydro-2,4,7-trimethyl-3-oxo-l,4(2H)-benzoxazin-6-yl)butyrate, oil;
methyl 4-oxo-4-(3,4-dihydro-4,6-dimethyl-3-oxo-l,4(2H)-benzoxazin-8-yl)butyrate, oil;
methyl 4-oxo-4-(3,4-dihydro-4,6-dimethyl-3-oxo-l,4(2H)-benzoxazin-7-yl)butyrate, oil;
methyl 4-oxo-4-(3,4-dihydro-2,4-trimethyl-3-oxo-l,4(2H)-benzoxazin-6-yl)butyrate, oil;
methyl, 4-oxo-4-(3,4-dihydro-4-methyl-3-oxo-l,4(2H)-benzoxazin-6-yl)-3-methylbutyrate, oil;
methyl 4-oxo-4-(3,4-dihydro-2,4-dimethyl-3-oxo-l,4(2H)-benzoxazin-6-yl)-3-methylbutyrate, oil;
methyl 4-oxo-4-(3,4-dihydro-4,7-dimethyl-3-oxo-l,4(2H)-benzoxazin-6-yl)-3-methylbutyrate, oil;
methyl 4 oxo-4-(3,4-dihydro-2,4,7-trimethyl-3-oxo-l,4(2H)-benzoxazin-6-yl)-3-methylbutyrate, oil;
methyl 4-oxo-4-(3,4-dihydro-4,6-dimethyl-3-oxo-l,4(2H)-benzoxazin-8-yl)3-methylbutyrate, oil; and
methyl 4-oxo-4-(3,4-dihydro-4-methyl-3-oxo-l,4(2H)-benzoxazin-7-yl)-3-methylbutyrate, oil.

EXAMPLE 6

4-Oxo-4-(3,4-dihydro-7-methyl-3-oxo-l,4(2H)-benzoxazin-6-yl)-3-methylbutyric acid

A. 3,4-Dihydro-7-methyl-3-oxo-l,4(2H)-benzoxazine was acylated with propionyl chloride by the method of Example 4 in 85% yield. The product of this operation was converted to the title compound as follows:

B. 3,4-Dihydro-7-methyl-6-(l-oxopropyl)-3-oxo-l,5(2H)-benzoxazine (23.6 g) was added to a mixture of l3 g of dimethylamine hydrochloride and l5 ml of 37% aqueous formaldehyde solution in 68 ml of acetic anhydride. After heating on a steam bath for three hours, 50 ml of acetone was added and heating was continued for l5 minutes. The solvents were removed by evaporation at reduced pressure and the residue was dissolved in lN HCl and and washed with ethyl acetate. The aqueous layer was basified with sodium hydroxide and the resultant crystals were collected by filtration. This product was dissolved in 500 ml of acetone and l0 ml of iodomethane were added. After heating at reflux overnight, the solid which formed was collected by filtration and washed with acetone. The product was dissolved in 400 ml of 50% aqueous methanol and l8 g of potassium cyanide in 200 ml of water was added. After stirring overnight at room temperature, the filter cake was suspended in 500 ml of 6N HCl and heated at reflux for l.5 hours. Upon cooling a white precipitate formed which was collected by filtration and washed with water to give l9.4 g (8l% yield) of the title compound, mp l69.5-l72°C.

The following compounds were prepared by the above procedure, using appropriate starting materials:
4-oxo-4-(3,4-dihydro-l,4(2H)-benzoxazin-6-yl)-3-methylbutyric acid;
4-oxo-4-(3,4-dihydro-2-methyl-l,4(2H)-benzoxazin-6-yl)-3-methylbutyric acid;
4-oxo-4-(3,4-dihydro-3-oxo-l,4(2H)-benzoxazin-6-yl)-3-methylbutyric acid;
4-oxo-4-(3,4-dihydro-2-methyl-3-oxo-l,4(2H)-benzoxazin-6-yl)-3-methylbutyric acid;
4-oxo-4-(3,4-dihydro-2,7-dimethyl-3-oxo-l,4(2H)-benzoxazin-6-yl)-3-methylbutyric acid;
4-oxo-4-(3,4-dihydro-4-(l-methylethyl)-2-methyl-3-oxo-l,4(2H)-benzoxazin-6-yl)-3-methylbutyric acid;
4-oxo-4-(3,4-dihydro-4-cyclopentyl-2-methyl-3-oxo-l,4(2H)-benzoxazin-6-yl)-3-methylbutyric acid;
4-oxo-4-(3,4-dihydro-6-methyl-3-oxo-l,4(2H)-benzoxazin-8-yl)-3-methylbutyric acid; and
4-oxo-4-(3,4-dihydro-6-methyl-l,4(2H)-benzoxazin-8-yl)-3-methylbutyric acid.

EXAMPLE 7

4-Oxo-4-(3,4-dihydro-3-oxo-l,4(2H)-benzoxazin-7-yl)-3-methylbutyric acid

3,4-Dihydro-7-(l-oxopropyl)-3-oxo-l,4(2H)-benzoxazine (from Example l) was converted to the title compound by the method of Example 6B.

EXAMPLE 8

6-(3,4-Dihydro-4-methanesulfonyl-l,4(2H)-benzoxazin-6-yl)-2,3,4,5-tetrahydropyridazin-3-one

Ethyl 4-oxo-4-(3,4-dihydro-4-methanesulfonyl-l,4-(2H)-benzoxazin-6-yl)butyrate was suspended in methanol and 2.2 equivalents of hydrazine was added. The mixture was brought to reflux and stirred for 24 hours. Upon cooling, crystals of the desired product formed and were collected by filtration. Recrystallization from ethanol gave pure title compound, mp 245°C.

Theor. $C_{13}H_{15}N_3O_4S$: C, 50.47; H, 4.90; N, l3.59
Found: C, 50.46; H, 4.85; N, l3.67

When in the above procedure, ethyl 4-oxo-4-(3,4-dihydro-4-methanesulfonyl-l,4(2H)-benzoxazin-6-yl)-3-ethylbutyrate; ethyl 4-oxo-4-(3,4-dihydro-4-methanesulfonyl-l,4(2H)-benzoxazin-6-yl)-3-hexylbutyrate; or ethyl 4-oxo-4-(3,4-dihydro-4-methanesulfonyl-l,4(2H)-benzoxazin-6-yl)-3-(l-methylethyl)butyrate is utilized as the starting material, the corresponding 5-ethyl-, 5-hexyl- or 5-(l-methylethyl)-pyridazin-3-one derivative is obtained.

EXAMPLE 9

6-(3,4-Dihydro-4-methanesulfonyl-l,4(2H)-benzoxazin-6-yl)-2,3,4,5-tetrahydro-2-methylpyridazin-3-one

6-(3,4-Dihydro-4-methanesulfonyl-l,4(2H)-benzoxazin-6-yl)-2,3,4,5-tetrahydropyridazin-3-one (3 g) was suspended in 50 ml of dimethylformamide and one equivalent of 60% sodium hydride in oil was added. when gas evolution ceased, one equivalent of methyl iodide was added and the mixture allowed to stand for l.5 hours followed by one hour at 40°C. The mixture was cooled and then poured into 200 ml of ice water, giving a precipitate that was collected by filtration, washed with water and recrystallized from ethanol. The material was further purified by chromatography on silica gel eluted with l:l EtOAc:$Et_2O$ yielding 0.97 g of the title product, mp l62-l65°C.

Theor. $C_{14}H_{17}N_3O_4S$: C, 5l.99; H, 5.3l; N, l3.00
Found: C, 5l.92; H, 5.32; N, l2.96

EXAMPLE l0

6-(3,4-Dihydro-4-methanesulfonyl-l,4(2H)-benzoxazin-6-yl)-2,3,4,5-tetrahydro-2-pentylpyridazine-3-one

6-(3,4-Dihydro-4-methanesulfonyl-l,4(2H)-benzoxazin-6-yl)-2,3,4,5-tetrahydropyridazin-3-one was reacted with pentyl bromide in place of methyl iodide following the procedure of Example 9. The title compound was recovered, yield l.46 g, mp l38-l39°C.

Theor. $C_{18}H_{25}N_3O_4S$: C, 56.96; H, 7.76; N, ll.07
Found: C, 56.67; H, 6.49; N, ll.05

When in the above procedure, bromocyclohexne or 2-bromopropane is utilized in place of pentyl bromide, the corresponding 2-cyclohexyl or 2-(l-methylethyl)pyridazinone is obtained.

EXAMPLE ll

6-(3,4-Dihydro-4-methanesulfonyl-l,4(2H)-benzoxazin-6-yl)-2,3,4,5-tetrahydro-2-(2-propenyl)pyridazin-3-one

6-(3,4-Dihydro-4-methanesulfonyl-l,4(2H)-benzoxazin-6-yl)-2,3,4,5-tetrahydropyridazin-3-one was reacted with allyl bromide instead of methyl iodide, following the procedure of Example 9. The title compound was recovered, yield 2.03 mg, mp l53-l55°C.

Theor. $C_{16}H_{19}N_3O_4S$: C, 54.99; H, 5.49; N, l2.03
Found: C, 54.95; H, 5.58; N, ll.92

EXAMPLE l2

6-(3,4-Dihydro-l,4(2H)-benzoxazin-6-yl)-2,3,4,5-tetrahydropyridazin-3-one

The method of Example 8 was followed using 4-oxo-4-(3,4-dihydro-l,4-(2H)-benzoxazin-6-yl)butyric acid as the starting material to give the title compound in 60% yield, mp l98-l99°C.

| | |
|---|---|
| Theor. $C_{12}H_{13}N_3O_2$ | C, 62.3l; H, 5.68; N, l8.l7 |
| Found: | C, 62.35; H, 5.72; N, l8.l8 |

EXAMPLE l3

6-(4-acetyl-3,4-dihydro-l,4(2H)-benzoxazin-6-yl)-2,3,4,5-tetrahydropyridazin-3-one

The method of Example 8 was followed using 4-oxo-4-(3,4-dihydro-4-acetyl-l,4(2H)-benzoxazin-6-yl) butyric acid as the starting material to yield the title compound in 40% yield, mp l56-l58°C.

| | |
|---|---|
| Theor. $C_{14}H_{15}N_3O_3$: | C, 6l.52; H, 5.54; N, l5.38 |
| Found: | C, 6l.49; H, 5.55; N, l5.24 |

EXAMPLE l4

6-(3,4-Dihydro-4-(3,4-dimethoxyphenylcarbonyl)-l,4(2H)-benzoxazin-6-yl)-2,3,4,5-tetrahydropyradazin-3-one

6-(3,4-Dihydro-l,4(2H)-benzoxazin-6-yl)-2,3,4,5-tetrahydropyridazin-3-one was dissolved in methylene chloride and l.l equivalent of triethylamine. l.l equivalent of 3,4-dimethoxybenzoylchloride was added and the mixture heated at reflux for four hours. The solution was washed with sodium bicarbonate solution and then evaporated to dryness. The residue was chromatographed on silica gel eluting with l:l ethyl acetate: ethyl ether. The title compound was collected as white needles, mp 207-208°C. compound in 40% yield, mp l56-l58°C.

| | |
|---|---|
| Theor. $C_{21}H_{21}N_3O_5$: | C, 63.78; H, 5.36; N, l0.63 |
| Found: | C, 63.78; H, 5.40; N, l0.64 |

EXAMPLE l5

6-(3,4-Dihydro-l,4(2H)-benzoxazin-6-yl)-2,3,4,5-tetrahydro-5-methylpyridazin-3-one

The method of Example 8 was followed using 4-oxo-4-(3,4-dihydro-l,4(2H)-benzoxazin-6-yl)-3-methyl-butyric acid as the starting material to product the title compound, mp l66-l68°C. compound in 40% yield, mp l56-l58°C.

| | |
|---|---|
| Theor. $C_{13}H_{15}N_3O_2$: | C, 63.65; H, 6.l8; N, l7.l3 |
| Found: | C, 63.47; H, 6.22; N, l6.90 |

When in the above procedure, 4-oxo-4-(3,4-dihydro-l,4(2H)-benzoxazin-6-yl)-3-ethylbutyric acid; 4-oxo-4-(3,4-dihydro-l,4(2H)-benzoxazin-6-yl)-3-hexylbutyric acid or 4-oxo-4-(3,4-dihydro-l,4(2H)-benzoxazin-6-yl)-3-(l-methylethyl)butyric acid is utilized, the corresponding 5-ethyl-, 5-hexyl- or 5-(l-methylethyl)-pyridazin-3-one derivative is obtained.

EXAMPLE l6

6-(4-acetyl-3,4-dihydro-l,4(2H)-benzoxazin-6-yl)-2,3,4,5-tetrahydro-5-methylpyridazine-3-one

6-(3,4-Dihydro-l,4(2H)-benzoxazin-6-yl)-2,3,4,5-tetrahydro-5-methylpyridazin-3-one was suspended in tetrahydrofuran and one equivalent of acetyl chloride was added. After one half hour at 0°C, the solvent was removed in vacuo, and the product was crystallized from ethanol in 6l% yield, mp l85.5-l86°C.

| | |
|---|---|
| Theor. $C_{15}H_{17}N_3O_3$: | C, 62.69; H, 5.97; N, l4.63 |
| Found: | C, 62.85; H, 6.03; N, l4.64 |

EXAMPLE l7

6-(3,4-Dihydro-4-methanesulfonyl-l,4(2H)-benzoxazin-6-yl)-2,3,4,5-tetrahydro-5-methylpyridazin-3-one

The method of Example l6 was followed using methanesulfonyl chloride instead of acetyl chloride. pyridine was added to the mixture. After one hour at 0°C, the mixture was warmed to room temperature and allowed to stir for 48 hours and then refluxed for 24 hours. Acetonitrile was added and the mixture was absorbed onto silica gel and eluted with ethyl acetate. The title compound was crystallized from ethanol to give a 25% yield, mp 207-2l2°C.

| | |
|---|---|
| Theor. $C_{l4}H_{l7}N_3O_4S$: | C, 5l.99; H, 5.3l; N, l3.00 |
| Found: | C, 52.42; H, 5.3l; H, l3.39 |

EXAMPLE l8

6-(3,4-Dihydro-2-methyl-l,4(2H)-benzoxazin-6-yl)-2,3,4,5-tetrahydropyridazin-3-one

The method of Example 8 was followed using 4-oxo-4-(3,4-dihydro-2-methyl-l,4(2H)benzoxazin-6-yl)-butyric acid as the starting material to give the desired product in l0% yield, mp 294.5-295.5°C.

| | |
|---|---|
| Theor. $C_{l3}H_{l5}N_3O_2$: | C, 63.65; H, 6.l8; N, l7.l3 |
| Found: | C, 63.37; H, 6.l6; N, l7.4l |

When in the above procedure, 4-oxo-4-(3,4-dihydro-2-methyl-7-pentyl-l,4(2H)-benzoxazin-6-yl)butyric acid;
4-oxo-4-(3,4-dihydro-2-hexyl-7-isopropyl-l,4(2H)-benzoxazin-6-yl)-2-hexylbutyric acid;
4-oxo-4-(3,4-dihydro-2-methyl-7-cyclohexyl-l,4(2H)-benzoxazin-6-yl)butyric acid;
4-oxo-4-(3,4-dihydro-2-isobutyl-7-methoxy-l,4(2H)-benzoxazin-6-yl)butyric acid or
4-oxo-4-(3,4-dihydro-2-cyclopentyl-l,4(2H)benzoxazin-6-yl)-butyric acid is used, the corresponding pyridazinone derivative is obtained.

EXAMPLE l9

6-(3,4-Dihydro-2-methyl-l,4(2H)-benzoxazin-6-yl)-2,3,4,5-tetrahydro-5-methylpyridazin-3-one

The method of Example 8 was followed using 4-oxo-4-(3,4-dihydro-2-methyl-l,4(2H)benzoxazin-6-yl)-3-methylbutyric acid as the starting material. The product was further purified by chromatography on silica gel, mp l79-l82°C.

| | |
|---|---|
| Theor. $C_{l4}H_{l7}N_3O_2$: | C, 64.83; H, 6.62; N, l6.2l |
| Found: | C, 64.5l; H, 6.64; N, l5.84 |

When in the above procedure, 4-oxo-4-(3,4-dihydro-2-methyl-l,4(2H)-benzoxazin-6-yl)-3-ethylbutyric acid;
4-oxo-4-(3,4-dihydro-2-methyl-l,4(2H)benzoxazin-6-yl)-3-hexylbutyric acid or 4-oxo-4-(3,4-dihydro-2-methyl-l,4(2H)-benzoxazin-6-yl)-3-(l-methylethyl)butyric acid is utilized, the corresponding 5-ethyl-, 5-hexyl- or 5-(l-methylethyl)- pyridizin-3-one derivative is obtained.

EXAMPLE 20

6-(3,4-Dihydro-3-oxo-l,4(2H)-benzoxazin-6-yl)-2,3,4,5-tetrahydropyridazin-3-one

Following the method of Example 8, but using methyl 4-oxo-4-(3,4-dihydro-3-oxo-l,4(2H)-benzoxazin-6-yl)butyrate, the title compound was obtained and was recrystallized from ethanol and then from acetonitrile as a hydrate, mp 274-275°C.

| | |
|---|---|
| Theor. $C_{l2}H_{ll}N_3O_3$ $H_2O$: | C, 57.70; H, 4.65; N, l6.83 |
| Found: | C, 57.54; H, 4.50; N, l6.79 |

EXAMPLE 2l

6-(3,4-Dihydro-4-methyl-3-oxo-l,4(2H)-benzoxazin-6-yl)-2,3,4,5-tetrahydropyridazin-3-one

The method of Example 8 was followed using methyl 4-oxo-4-(3,4-dihydro-4-methyl-3-oxo-l,4(2H)benzoxazin-6-yl)-butyrate as the starting material. The product was purified by column chromatography followed by several recrystallizations from acetonitrile, mp 247-247.5°C.

| | |
|---|---|
| Theor. $C_{13}H_{13}N_3O_3$: | C, 60.2l; H, 5.06; N, l6.2l |
| Found: | C, 59.85; H, 4.98; N, l6.26 |

EXAMPLE 22

6-(3,4-Dihydro-3-oxo-l,4(2H)-benzoxazin-6-yl)-2,3,4,5-tetrahydro-5-methylpyridazin-3-one

The method of Example 8 was followed using methyl 4-oxo-4-(3,4-dihydro-3-oxo-l,4(2H)benzoxazin-6-yl)-3-methylbutyrate as the starting material. The product was purified by crystallization from acetonitrile, followed by column chromatography on silica gel and eluted with 5% methanol in dichloromethane, mp 265-267°C.

| | |
|---|---|
| Theor. $C_{13}H_{13}N_3O_3$ l/4$H_2O$: | C, 59.l9; H, 5.l7; N, l5.93 |
| Found: | C, 59.22; H, 4.98; N, l5.92 |

When in the above procedure, 4-oxo-4-(3,4-dihydro-3-oxo-l,4(2H)-benzoxazin-6-yl)-3-ethylbutyrate; 4-oxo-4-(3,4-dihydro-3-oxo-l,4(2H)-benzoxazin-6-yl)-3-hexylbutyrate or 4-oxo-4-(3,4-dihydro-3-oxo-l,4(2H)benzoxazin-6-yl)-3-(l-methylethyl)butyrate is utilized, the corresponding 5-ethyl-, 5-hexyl- or 5-(l-methylethyl)-pyridazin-3-one derivative is obtained.

EXAMPLE 23

6-(3,4-Dihydro-4-methyl-3-oxo-l,4(2H)-benzoxazin-6-yl)-2,3,4,5-tetrahydro-5-methylpyridazin-3-one

The method of Example 8 was followed using methyl 4-oxo-4-(3,4-dihydro-4-methyl-3-oxo-l,4(2H)-benzoxazin-6-yl)-3-methylbutyrate as the starting material. The product was purified by chromatography and eluted with 5% methanol in dichloromethane, mp 2l5-2l8°C.

| | |
|---|---|
| Theor. $C_{14}H_{14}N_3O_3$: | C, 6l.52; H, 5.54; N, l5.34 |
| Found: | C, 6l.80; H, 5.75; N, l5.63 |

EXAMPLE 24

6-(3,4-Dihydro-2-methyl-3-oxo-l,4(2H)-benzoxazin-6-yl)-2,3,4,5-tetrahydropyridazin-3-one

The method of Example 8 was followed using 4-oxo-4-(3,4-dihydro-2-methyl-3-oxo-l,4(2H)-benzoxazine-6-yl)butyric acid as the starting material, to produce the title compound in 75% yield, mp 275-276°C.

| | |
|---|---|
| Theor. $C_{13}H_{13}N_3O_3$: | C, 60.2l; H, 5.06; N, l6.2l |
| Found: | C, 60.02; H, 5.22; N, l6.08 |

EXAMPLE 25

6-(3,4-Dihydro-2,4-dimethyl-3-oxo-l,4(2H)-benzoxazin-6-yl)-2,3,4,5-tetrahydropyridazin-3-one

The title compound was produced in 25% yield, by following the method of Example 8, using methyl 4-oxo-4-(3,4-dihydro-2,4-dimethyl-oxo-l,4(2H)-benzoxazin-6-yl)-butyrate as the starting material, mp 2l0-2ll°C.

| | |
|---|---|
| Theor. $C_{14}H_{15}N_3O_3$ l/2$H_2O$: | C, 59.56; H, 5.72; N, l5.l6 |
| Found: | C, 59.93; H, 5.48; N, l5.l6 |

EXAMPLE 26

6-(3,4-Dihydro-2-methyl-3-oxo-l,4(2H)-benzoxazin-6-yl)-2,3,4,5-tetrahydro-5-methylpyridazin-3-one

The method of Example 8 was followed using 4-oxo-4-(3,4-dihydro-2-methyl-3-oxo-l,4(2H)-benzoxazin-6-yl)-3-methylbutyric acid as the starting material, to yield the title compound in 50% yield, mp 27l-272°C.

| | |
|---|---|
| Theor. $C_{14}H_{15}N_3O_3$: | C, 6l.52; H, 5.54; N, l5.38 |
| Found: | C, 6l.34; H, 5.59; N, l5.4l |

When in the above procedure, 4-oxo-4-(3,4-dihydro-2-methyl-7-pentyl-3-oxo-l,4(2H)-benzoxazin-6-yl)butyric acid; 4-oxo-4-(3,4-dihydro-2-hexyl-7-isopropyl-3-oxo-l,4(2H)-benzoxazin-6-yl)butyric acid; 4-oxo-4-(3,4-dihydro-2-methyl-7-cyclohexyl-3-oxo-l,4(2H)-benzoxazin-6-yl)butyric acid; 4-oxo-4-(3,4-dihydro-2-isobutyl-7-methoxy-l,4(2H)-benzoxazin-6-yl)butyric acid or 4-oxo-4-(3,4-dihydro-2-cyclopentyl-3-oxo-l,4(2H)benzoxazin-6--yl)butyric acid is used, the corresponding pyridazinone derivative is obtained.

EXAMPLE 27

6-(3,4-Dihydro-2,4-dimethyl-3-oxo-l,4(2H)-benzoxazin-6-yl)-2,3,4,5-tetrahydro-5-methylpyridazin-3-one

Following the method of Example 8, using methyl 4-oxo-4-(3,4-dihydro-2,4-dimethyl-3-oxo-l,4(2H)-benzoazin-6-yl)-3-methylbutyrate as the starting material, the title compound was obtained, in 40% yield, mp l84-l85°C.

| | |
|---|---|
| Theor. $C_{15}H_{17}N_3O_3$: | C, 62.70; H, 5.98; N, l4.63 |
| Found: | C, 62.75; H, 5.95; N, l4.79 |

EXAMPLE 28

6-(3,4-Dihydro-7-methyl-3-oxo-l,4(2H)-benzoxazin-6-yl)-2,3,4,5-tetrahydropyridazin-3-one

The method of Example 8 was followed, using 4-oxo-4-(3,4-dihydro-7-methyl-3-oxo-l,4(2H)-benzoxazin-6-yl) butyric acid as the starting material, to produce the title compounds in 55% yield, mp 255-257°C.

| | |
|---|---|
| Theor. $C_{13}H_{13}N_3O_3$: | C, 60.2l; H, 5.06; N, l6.2l |
| Found: | C, 59.90; H, 5.26; N, l5.95 |

When in the above procedure, 4-oxo-4-(3,4-dihydro-7-pentyl-3-oxo-l,4(2H)-benzoxazin-6-yl)butyric acid; 4-oxo-4-(3,4-dihydro-2-hexyl-7-isopropyl-3-oxo-l,4(2H)-benzoxazin-6-yl)butyric acid; 4-oxo-4-(3,4-dihydro-7-cyclohexyl-3-oxo-l,4(2H)-benzoxazin-6-yl)butyric acid; 4-oxo-4-(3,4-dihydro-2-isobutyl-7-methoxy-3-oxo-l,4(2H)-benzoxazin-6-yl)-butyric acid or 4-oxo-4-(3,4-dihydro-2-cyclopentyl-3-oxo-l,4(2H)-benzoxazin-6-yl)butyric acid is used, the corresponding pyridazinone derivative is obtained.

EXAMPLE 29

6-(3,4-Dihydro-4,7-dimethyl-3-oxo-l,4(2H)-benzoxazin-6-yl)-2,3,4,5-tetrahydropyridazin-3-one

The title compound was obtained, in 47% yield, by following the method of Example 8, using methyl 4-oxo-4-(3,4-dihydro-4,7-dimethyl-3-oxo-l,4(2H)-benzoxazin-6-yl)-butyrate as the starting material, mp 227-228.5°C.

| | |
|---|---|
| Theor. $C_{14}H_{15}N_3O_3$: | C, 6l.52; H, 5.54; N, l5.38 |
| Found: | C, 6l.65; H, 5.57; N, l5.26 |

EXAMPLE 30

6-(3,4-Dihydro-7-methyl-3-oxo-l,4(2H)-benzoxazin-6-yl)-2,3,4,5-tetrahydro-5-methylpyridazin-3-one

The method of Example 8 was followed, using 4-oxo-4-(3,4-dihydro-7-methyl-3-oxo-l,4(2H)-benzoxazin-6-yl)-3-methylbutyric acid as the starting material to give the title compound in 5l% yield, mp l63-l66°C.

| | |
|---|---|
| Theor. $C_{14}H_{15}N_3O_3$ l/4$H_2O$: | C, 60.52; H, 5.63; N, l5.l3 |
| Found: | C, 60.65; H, 5.62; N, l5.03 |

EXAMPLE 3l

6-(3,4-Dihydro-4,7-dimethyl-3-oxo-l,4(2H)-benzoxazin-6-yl)-2,3,4,5-tetrahydro-5-methylpyridazin-3-one

Following the method of Example 8, using 4-oxo-4-(3,4-dihydro-4,7-dimethyl-3-oxo-l,4(2H)-benzoxazin-6-yl)-3-methylbutyrate as the starting material, the title compound was produced, mp l80-l82°C.

| | |
|---|---|
| Theor. $C_{15}H_{17}N_3O_3$: | C, 62.70; H, 5.98; N, l4.63 |
| Found: | C, 62.77; H, 6.06; N, l4.57 |

EXAMPLE 32

6-(3,4-Dihydro-2,7-dimethyl-3-oxo-l,4(2H)-benzoxazin-6-yl)-2,3,4,5-tetrahydropyridazin-3-one

The method of Example 8 was followed, using 4-oxo-4-(3,4-dihydro-2,7-dimethyl-3-oxo-l,4(2H)-benzoxazin-6-yl)butyric acid to give the title compound, mp 252-254°C.

| | |
|---|---|
| Theor. $C_{14}H_{15}N_3O_3$ l/4$H_2O$: | C, 60.52; H, 5.64; N, l5.l3 |
| Found: | C, 60.50; H, 5.45; N, l5.63 |

EXAMPLE 33

6-(3,4-Dihydro-2,4,7-trimethyl-3-oxo-l,4(2H)-benzoxazin-6-yl)-2,3,4,5-tetrahydropyridazin-3-one

Following the method of Example 8, using methyl 4-oxo-4-(3,4-dihydro-2,4,7-trimethyl-3-oxo-l,4(2H)-benzoxazin-6-yl)butyrate as the starting material, the title compound was obtained, mp 2l0-2l2°C.

| | |
|---|---|
| Theor. $C_{15}H_{17}N_3O_3$: | C, 62.70; H, 5.98; N, l4.63 |
| Found: | C, 62.85; H, 6.ll; N, l4.93 |

EXAMPLE 34

6-(3,4-Dihydro-2,7-dimethyl-3-oxo-l,4(2H)-benzoxazin-6-yl)-2,3,4,5-tetrahydro-5-methylpyridazin-3-one

The method of Example 8 was followed, using 4-oxo-4-(3,4-dihydro-2,7-dimethyl-3-oxo-l,4(2H)-benzoxazin-6-yl)-3-methylbutyric acid, to yield the title compound, mp l90-l9l°C.

| | |
|---|---|
| Theor. $C_{15}H_{17}N_3O_3$ l/2$H_2O$: | C, 60.80; H, 6.l4; N, l4.l8 |
| Found: | C, 6l.l8; H, 6.42; N, l3.78 |

EXAMPLE 35

6-(3,4-Dihydro-2,4,7-trimethyl-3-oxo-l,4(2H)-benzoxazin-6-yl)-2,3,4,5-tetrahydro-5-methylpyridazine-3-one

Following the method of Example 8, using methyl 4-oxo-4-(3,4-dihydro-2,4,7-trimethyl-3-oxo-l,4(2H)-benzoxazin-6-yl)-3-methylbutyrate as the starting material, the title compound was produced, mp l90-l92°C.

| | |
|---|---|
| Theor. $C_{16}H_{19}N_3O_3$ l/2$H_2O$: | C, 6l.9l; H, 6.5l; N, l3.54 |
| Found: | C, 62.02; H, 6.52; N, l3.86 |

When in the above procedure, 4-oxo-4-(3,4-dihydro-2,4-dimethyl-7-pentyl-3-oxo-l,4(2H)-benzoxazin-6-yl)-3-methylbutyrate;4-oxo-4-(3,4-dihydro-2-hexyl-4-methyl-7-isopropyl-3-oxo-l,4(2H)-benzoxazin-6-yl)-3-m ethylbutyrate; 4-oxo-(3,4-dihydro-2,4-dimethyl-7-cyclohexyl-3-oxo-l,4(2H)-benzoxazin-6-yl)-3-methylbutyrate; 4-oxo-4-(3,4-dihydro-2-isobutyl-4-methyl-7-methoxy-3-oxo-l,4(2H)-benzoxazin-6-yl)-3-methylbutyrate; or 4-oxo-4-(3,4-dihydro-2-cyclopentyl-4,7-dimethyl-3-oxo-l,4(2H)-benzoxazin-6-yl)-3-methylbutyrate is used, the corresponding pyridazinone derivative is obtained.

EXAMPLE 36

6-(3,4-Dihydro-2-methyl-4-(l-methylethyl)-3-oxo-l,4(2H)-benzoxazin-6-yl)-2,3,4,5-tetrahydro-5-methylpyridazin-3-one

The method of Example 8 was followed, using 4-oxo-4-(3,4-dihydro-4-(l-methylethyl)-3-oxo-l,4(2H)-benzoxazin-6-yl)-3-methylbutyric acid as the starting material to give the title compound, mp 204-205°C.

| | |
|---|---|
| Theor. $C_{17}H_{21}N_3O_3$: | C, 64.73; H, 6.72; N, l3.33 |
| Found: | C, 64.67; H, 6.66; N, l3.42 |

EXAMPLE 37

6-(3,4-Dihydro-4-cyclopentyl-2-methyl-3-oxo-l,4(2H)-benzoxazin-6-yl)-2,3,4,5-tetrahydro-5-methylpyridazin-3-one

Following the method of Example 8, using 4-oxo-4-(3,4-dihydro-4-cyclopentyl-2-methyl-3-oxo-l,4(2H)-

benzoxazin-6-yl)-3-methylbutyric acid as the starting material, the title compound was obtained, mp 220-223°C.

| | |
|---|---|
| Theor. $C_{19}H_{23}N_3O_3$: | C, 66.84; H, 6.80; N, I2.3I |
| Found: | C, 66.6I; H, 6.78; N, I2.29 |

EXAMPLE 38

6-(3,4-Dihydro-6-methyl-3-oxo-l,4(2H)-benzoxazin-8-yl)-2,3,4,5-tetrahydropyridazin-3-one

The method of Example 8 was followed, using 4-oxo-4(3,4-dihydro-6-methyl-3-oxo-l,4(2H)-benzoxazin-8-yl)butyric acid as the starting material to give the title compound, mp 266-270°C.

| | |
|---|---|
| Theor. $C_{13}H_{14}N_3O_3$: | C, 60.52; H, 5.06; N, I6.2I |
| Found: | C, 60.I3; H, 5.26; N, I6.28 |

EXAMPLE 39

6-(3,4-Dihydro-4,6-dimethyl-3-oxo-l,4(2H)-benzoxazin-8-yl)-2,3,4,5-tetrahydropyridazin-3-one

Following the method of Example 8, using methyl 4-oxo-4-(3,4-dihydro-4,6-dimethyl-3-oxo-l,4(2H)-benzoxazin-8-yl)butyrate as the starting material, the title compound was obtained in I6% yield, mp 266-270°C

EXAMPLE 40

6-(3,4-Dihydro-6-methyl-3-oxo-l,4(2H)-benzoxazin-8-yl)-2,3,4,5-tetrahydro-5-methylpyridazin-3 one

The method of Example 8 was followed, using 4-oxo-4-(3,4-dihydro-6-methyl-3-oxo-l,4(2H)-benzoxazin-8-yl) butyric acid as the starting material, to give the title compound in 3I% yield, mp 252-253.5°C.

| | |
|---|---|
| Theor. $C_{14}H_{15}N_3O_3$: | C, 6I.52; H, 5.54; N, I5.38 |
| Found: | C, 6I.II; H, 5.68; N, I5.26 |

EXAMPLE 4I

6-(3,4-Dihydro-4,6-dimethyl-3-oxo-l,4(2H)-benzoxazin-8-yl)-2,3,4,5-tetrahydro-5-methylpyridan-3-one

The method of Example 8 was followed, using methyl 4-oxo-4-(3,4-dihydro-4,6-dimethyl-3-oxo-l,4(2H)-benzoxazin-8-yl)-3-methylbutyrate as the starting material, to product the title compound in I5% yield after column chromatography on silica gel eluted with 5% methanol in dichloromethane, mp 2I2-2I3°C.

| | |
|---|---|
| Theor. $C_{15}H_{17}N_3O_3$: | C, 62.69; H, 5.98; N, I4.63 |
| Found: | C, 62.27; H, 5.92; N, I4.57 |

EXAMPLE 42

6-(3,4-Dihydro-6-methyl-l,4(2H)-benzoxazin-8-yl)-2,3,4,5-tetrahydro-5-methylpyridazin-3-one

Following the method of Example 8, using ethyl 4-oxo-4-(3,4-dihydro-6-methyl-l,4(2H)-benzoxazin-8-yl)-3-methylbutyrate as the starting material, the title compound was prepared in 60% yield, mp I60-I62°C.

| | |
|---|---|
| Theor. $C_{14}H_{17}N_3O_3$: | C, 64.83; H, 6.62; N, I6.2I |
| Found: | C, 64.87; H, 6.66; N, I6.3I |

When in the above procedure, 4-oxo-4-(3,4-dihydro-6-methyl-l,4(2H)-benzoxazin-8-yl)-3-ethylbutyrate; 4-oxo-4-(3,4-dihydro-6-methyl-l,4(2H)-benzoxazin-8-yl-3-hexylbutyrate or 4-oxo-4-(3,4-dihydro-6-methyl-l,4 (2H)-benzoxazin-8-yl)-3-(l-methylethyl)butyrate is utilized, the corresponding 5-ethyl-, 5-hexyl- or 5-(l-methylethyl)-pyridazin-3-one derivative is obtained.

EXAMPLE 43

6-(3,4-Dihydro-4,6-dimethyl-3-oxo-l,4(2H)-benzoxazin-7-yl)-2,3,4,5-tetrahydropyridazin-3-one

The method of Example 8 was followed, using methyl 4-oxo-4-(3,4-dihydro-4,6-dimethyl-3-oxo-l,4(2H)-benzoxazin-7-yl)butyrate as the starting material to produce the title compound, mp 2II-2I3°C.

| | |
|---|---|
| Theor. $C_{14}H_{15}N_3O_3$: | C, 61.52; H, 5.54; N, 15.38 |
| Found: | C, 61.57; H, 5.49; N, 15.28 |

EXAMPLE 44

6-(3,4-Dihydro-2,2-dimethyl-3-oxo-1,4(2H)-benzoxazin-6-yl)-2,3,4,5-tetrahydropyridazin-3-one

The method of Example 8 was followed, using 4-oxo-4-(3,4-dihydro-2,2-dimethyl-3-oxo-1,4(2H)-benzoxazin-6-yl) butyric acid as the starting material, to give the title compound, mp 251-254°C.

| | |
|---|---|
| Theor. $C_{14}H_{15}N_3O_3$: | C, 61.52; H, 5.54; N, 15.38 |
| Found: | C, 61.40; H, 5.58; N, 15.74 |

EXAMPLE 45

6-(3,4-Dihydro-2,2,4-trimethyl-3-oxo-1,4(2H)-benzoxazin-6-yl)-2,3,4,5-tetrahydropyridazin-3-one

Following the method of Example 8, using methyl 4-oxo-4-(3,4-dihydro-2,2,4-trimethyl-3-oxo-1,4(2H)-benzoxazin-6-yl)butyrate, the title compound was produced, mp 169-171°C.

| | |
|---|---|
| Theor. $C_{15}H_{17}N_3O_3$: | C, 62.69; H, 5.98; N, 14.63 |
| Found: | C, 62.79; H, 5.86; N, 14.40 |

EXAMPLE 46

6-(3,4-Dihydro-3-oxo-1,4(2H)-benzoxazin-7-yl)-2,3,4,5-tetrahydro-5-methylpyridazin-3-one

The method of Example 8 was followed, using 4-oxo-4-(3,4-dihydro-3-oxo-1,4(2H)-benzoxazin-7-yl)-3-methylbutyric acid as the starting material. The title compound was obtained and recrystallized from dimethylformamide-water, then ethanol, mp > 300°C.

| | |
|---|---|
| Theor. $C_{13}H_{13}N_3O_3$ $1/4H_2O$: | C, 59.18; H, 5.17; N, 15.93 |
| Found: | C, 58.88; H, 5.04; N, 16.03 |

When in the above procedure, 4-oxo-4-(3,4-dihydro-3-oxo-1,4(2H)-benzoxazin-7-yl)-3-ethylbutyrate; 4-oxo-4-(3,4-dihydro-3-oxo-1,4(2H)-benzoxazin-7-yl)-3-hexylbutyrate or 4-oxo-4-(3,4-dihydro-3-oxo-1,4(2H)-benzoxazin-7-yl)-3-(1-methylethyl)butyrate is utilized, the corresponding 5-ethyl-, 5-hexyl- or 5-(1-methylethyl)-pyridazin-3-one derivative is obtained.

EXAMPLE 47

6-(3,4-Dihydro-4-methyl-3-oxo-1,4(2H)-benzoxazin-7-yl)-2,3,4,5-tetrahydro-5-methylpyridazin-3-one

The method of Example 8 was followed, using methyl 4-oxo-4-(3,4-dihydro-4-methyl-3-oxo-1,4(2H)-benzoxazin-7-yl)-3-methyl-butyrate. The product was purified by chromatography on silica gel eluted with 5% $CH_3OH$ in $CH_2Cl_2$, mp 188-190°C.

| | |
|---|---|
| Theor. $C_{14}H_{15}N_3O_3$: | C, 61.52; H, 5.54; N, 15.38 |
| Found: | C, 61.45; H, 5.68; N, 15.15 |

EXAMPLE 48

6-(3,4-Dihydro-2-methyl-3-oxo-1,4(2H)-benzoxazin-7-yl)-2,3,4,5-tetrahydropyridazin-3-one

The method of Example 8 was followed, using 4-oxo-4-(3,4-dihydro-2-methyl-3-oxo-1,4(2H)-benzoxazin-7-yl)butyric acid. The product was purified by column chromatography on silica gel eluted with 5% $CH_3OH$ in $CH_2Cl_2$. Trituration with water gave the product as a hydrate, mp 294-295°C.

| | |
|---|---|
| Theor. $C_{13}H_{13}N_3O_3$ $1/4H_2O$: | C, 59.18; H, 5.15; N, 15.93 |
| Found: | C, 59.15; H, 4.93; N, 15.83 |

The corresponding 2-alkyl-pyridazinone derivatives of the compounds prepared in any of the preceding examples are prepared in accordance with the procedures of Examples 9 and 10. The corresponding 4-acyl, 4-arylacyl or 4-alkane sulfonylbenzoxazinyl derivatives of the compounds prepared in the preceding examples where $R_3$ = H are prepared in accordance with the procedures of Examples 14, 16 and 17.

EXAMPLE 49

4-(3,4-Dihydro-3-oxo-l,4[2H]-benzothiazin-6-yl)-4-oxobutyric acid

To l00 g of aluminum chloride was added l5.6 ml of dimethylformamide. To the hot slurry was added an intimate mixture of l7 g of 3,4-dihydro-3-oxo-l,4[2H]-benzothiazine and l0 g of succinic anhydride. After l5 minutes at 75°C, the mixture was poured into 600 ml of ice and the precipitate collected by filtration washed with water then acetone to give 20 g (73% yield) of named compound decomposing at 2l5°-2l8°C.

Theor. $C_{12}H_{11}NO_4S$ l/2$H_2O$: C, 59.l8; H, 5.l5; N, l5.93
Found: C, 59.l5; H, 4.93; N, l5.83

The corresponding 2-alkyl-pyridazinone derivatives of the compounds prepared in any of the preceding examples are prepared in accordance with the procedures of Examples 9 and l0. The corresponding 4-acyl, 4-arylacyl or 4-alkane sulfonylbenzoxazinyl derivatives of the compounds prepared in the preceding examples where $R_3$ = H are prepared in accordance with the procedures of Examples l4, l6 and l7.

EXAMPLE 50

4-(3,4-Dihydro-3-oxo-l,4[2H]-benzothiazin-6-yl)-4-oxobutyric acid

To l00 g of aluminum chloride was added l5.6 ml of dimethylformamide. To the hot slurry was added an intimate mixture of l7 g of 3,4-dihyro-3-oxo-l,4[2H]-benzothiazine and l0 g of succinic anhydride. After l5 minutes at 75°C, the mixture was poured into 600 ml of ice and the precipitate collected by filtration and washed with water then acetone to give 20 g (73% yield) of named compound decomposing at 2l5°-2l8°C.

Theor. $C_{12}H_{11}NO_4S$ l/2 $H_2O$: C, 52.54; H, 4.42; N, 5.ll
Found: C, 52.54; H, 4.20; N, 5.l8

The following compounds are prepared by the above procedure, using the appropriate starting materials:
4-(3,4-dihydro-2-methyl-3-oxo-l,4[2H]-benzothiazin-6-yl)-4-oxobutyric acid and
4-(3,4-dihydro-2,2-dimethyl-3-oxo-l,4[2H]-benzothiazin-6-yl)-4-oxobutyric acid.

EXAMPLE 5l

Methyl 4-(3,4-dihydro-4-methyl-3-oxo-l,4[2H]-benzothiazin-6-yl)-4-oxobutyrate

4-(3,4-Dihydro-3-oxo-l,4[2H]-benzothiazin-6-yl)-4-oxobutyric acid (3 g) was suspended in 50 ml of dimethylformamide and 2.2 equivalents of 60% sodium hydride (l.0 g) were added. After 30 minutes at room temperature, 2.2 equivalents of methyl iodide (3.5 g) were added and the mixture stirred overnight. The mixture was poured into ice water and the precipitate collected by filtration. The solid was characterized by lH NMR and used in the next reaction (see Example 54).

The following compounds are prepared by the above procedure, using the appropriate starting materials:
Methyl 4-(3,4-dihydro-2-methyl-3-oxo-l,4[2H]-benzothiazin-6-yl)-4-oxobutyrate, and
Methyl 4-(3,4-dihydro-2,2-dimethyl-3-oxo-l,4[2H]-benzothiazin-6-yl)-4-oxobutyrate.

EXAMPLE 52

4-(3,4-Dihydro-3-oxo-l,4[2H]-benzothiazin-7-yl)-3-methyl-4-oxobutyric acid

3,4-Dihydro-3-oxo-7-(l-oxopropyl)-l,4[2H]-benzothiazine (5.0 g) was added to a mixture of 2.25 ml of formalin solution and 2.72 g of dimethylamine hydrochloride in 7.5 ml of acetic anhydride and heated to l00°C overnight. Acetone (20 ml) was added and the mixture heated at reflux for l5 minutes before the volitiles were removed at reduced pressure. The residue was taken up in lN HCl and washed with ethyl acetate. The aqueous portion was chilled in ice and basified with l2.5 N NaOH. The basic solution was extracted with ethyl acetate and dried over sodium sulfate. The solvent was removed and the residue taken up in acetone and 3 ml of methyl iodide added. The mixture was heated at reflux for three hours, then cooled, and the precipitate collected by filtration and washed with acetone to give 6.6 g of the quaternary ammonium salt of the mannich product.

The quaternary salt (6.6 g) was dissolved in 20% methanol/water and 3.8 g of potassium cyanide in 30 ml of water and added. The mixture was stirred at room temperature for 48 hours and the resultant precipitate collected by suction filtration to give 4-(3,4-dihydro-3-oxo-l,4[2H]-benzothiazin-7-yl)-3-methyl-4-oxo-butyro-nitrile. The nitrile was heated to reflux in 200 ml of 6N HCl for 30 minutes, then cooled and diluted with an equal

volume of ice water. The solid collected was used in Example 55.

EXAMPLE 53

Methyl 4-(3,4-dihydro-4-methyl-3-oxo-l,4[2H]-benzothiazin-7-yl)-3-methyl-4-oxobutyrate

4-(3,4-Dihydro-3-oxo-l,4[2H]-benzothiazin-7-yl)-3-methyl-4-oxobutyric acid (l.7 g ) was dissolved in 50 ml of dimethylformamide and 0.23 g of 60% sodium hydride was added. After 30 minutes, 0.7 ml of methyl iodide was added and the mixture stirred at room temperature for three hours, then poured into l00 ml of ice water and extracted with ethyl acetate. The organic layer was washed with brine and dried over sodium sulfate and evaporated. The residue was chromatographed on a silica gel column eluted with 5% methanol in methylene chloride. The solvent was removed and the oil obtained was used in Example 56.

EXAMPLE 54

6-(3,4-Dihydro-3-oxo-l,4[2H]-benzothiazin-6-yl)-2,3,4,5-tetrahydropyridazin-3-one

4-(3,4-Dihydro-3-oxo-l,4[2H]-benzothiazin-6-yl)-4-oxobutyric acid (2.0 g) was suspended in 60 ml of ethanol and l ml of hydrazine added. The mixture was heated at reflux for three hours then cooled slowly. Filtration gave l.85 g of the title compounds, mp 299°-203°C.

| | |
|---|---|
| Theor. $C_{l2}H_{ll}N_3O_2S$ l/2 $H_2O$: | C, 53.32; H, 4.48; N, l5.55 |
| Found: | C, 53.02; H, 4.26; N, l5.94 |

The following compounds are prepared by the above procedure, using the appropriate starting materials:
4-(3,4-dihydro-2-methyl-3-oxo-l,4[2H]-benzothiazin-6-yl)-4-oxobutyric acid, and
4-(3,4-dihydro-2,2-dimethyl-3-oxo-l,4[2H]-benzothiazin-6-yl)-4-oxobutyric acid.

EXAMPLE 55

6-(3,4-Dihydro-4-methyl-3-oxo-l,4[2H]-benzothiazin-6-yl)-2,3,4,5-tetrahydropyridazin-3-one

Methyl 4-(3,4-dihydro-4-methyl-3-oxo-l,4[2H]-benzothiazin-6-yl)-4-oxobutyrate (2.5 g) was heated at reflux in 70 ml of ethanol containing l ml of hydrazine for three hours. Filtration gave l.5 g of the named compound, melting at 24l°-242°C.

| | |
|---|---|
| Theor. $C_{l3}H_{l3}N_3O_3S$ l/4 $H_2O$: | C, 55.80; H, 4.88; N, l5.02 |
| Found: | C, 55.64; H, 4.87; N, l5.06 |

The following compounds are prepared by the above procedure, using the appropriate starting materials:
4-(3,4-dihydro-2,4-dimethyl-3-oxo-l,4[2H]-benzothiazin-5-yl)-4-oxobutyric acid, and
4-(3,4-dihydro-2,2,4-trimethyl-3-oxo-l,4[2H]-benzothiazin-6-yl)-4-oxobutyric acid.

EXAMPLE 56

6-(3,4-Dihydro-3-oxo-l,4[2H]-benzothiazin-7-yl)-2,3,4,5-tetrahydro-5-methylpyridazin-3-one

4-(3,4-Dihydro-3-oxo-l,4[2H]-benzothiazin-7-yl)-4-oxo-3-methylbutyric acid (l.2 g) was heated at reflux overnight in 50 ml of ethanol containing 0.2 ml of hydrazine. Filtration gave a white powder which was chromatographed on silica gel eluted with 5% methanol in methylene chloride, yielding l80 mg of the title compound melting > 300°C. dimethylformamide-water, then ethanol, mp > 300°C.

| | |
|---|---|
| Theor. $C_{l3}H_{l3}N_3O_3S$ l/4 $H_2O$: | C, 55.80; H, 4.88; N, l5.02 |
| Found: | C, 56.l3; H, 4.75; N, l5.00 |

EXAMPLE 57

6-(3,4-Dihydro-3-oxo-l,4[2H]-benzothiazin-7-yl)-2,3,4,5-tetrahydro-5-methylpyridazin-3-one

Methyl 4-(3,4-dihydro-4-methyl-3-oxo-l,4(2H)-benzothiazin-7-yl)-4-oxo-3-methylbutyrate (l.7 g) was heated at reflux overnight with 0.3 ml of hydrazine in 50 ml of ethanol. Filtration gave a white powder which was chromatographed on silica gel eluted with 5% methanol in methylene chloride, yielding ll0 mg of the named compound, mp l93°-l94.5°C.

| | |
|---|---|
| Theor. $C_{14}H_{15}N_3O_3S$: | C, 58.10; H, 5.24; N, 14.52 |
| Found: | C, 57.81; H, 5.42; N, 14.15 |

EXAMPLE 58

3,4-Dihydro-3-oxo-6-oxopropyl-l,4[2H]benzothiazine

4-Amino-3-thiocycano to propriophenone (prepared by the method of K. D. Luess and R. Pohloudek-Fabini in Arch. Pharm., 299(10), 878-884 (1966)) (35 g) was heated to reflux in 250 ml of water containing 90 g of sodium sulfide nonahydrate. The solution was cooled to room temperature and 60 ml of acetic acid was added. The precipitate that formed was collected and washed with water giving the mercapto compound.

4-Amino-3-mercaptopropiophenone was stirred in 200 ml of water containing 8.5 g of sodium hydroxide. The resultant mixture was filtered and 22 g of sodium chloroacetate added to the filtrate in 150 ml of water. After 30 minutes, 4 ml of acetic acid was added and the mixture heated to reflux. After 10 minutes, heat was removed and the mixture stirred at room temperature overnight. The yellow precipitate was collected by filtration and washed with water to give, after drying, 18.5 g of benzothiazine melting at 215°-220°C.

4-(3,4-Dihydro-3-oxo-l,4[2H]benzothiazin-7-yl)-4-oxobutyric acid was prepared by the same method as above. The starting material for this preparation was 4-aminophenyl-4-oxobutyric acid which is described by Thyes in J. Med. Chem., 26, 800 (1983). Thiocyanation was carried out by the method of K. D. Luess and R. Pohloudek-Fabini in Arch. Pharm., 299(10), 878-882 (1966).

EXAMPLE 59

Methyl 4-(3,4-dihydro-3-oxo-l,4(2H)-benzoxazin-7-yl)-3-methyl-4-oxobutyrate

To a stirred slurry of 4-(3,4-dihydro-3-oxo-l,4(2H)-benzoxazin-7-yl)-3-methyl-4-oxobutyric acid (131.63 g, 0.5 mol) in methanol (1.25 l) at 20°C was added hydrogen chloride gas (ca. 25 g) over a 30 min period. The temperature rose to 30°C and the slurry cleared to give a brown solution. Following stirring at 25°C for 1 hour a precipitate formed. The resulting slurry was cooled to 5°C and stirred an additional 2 hours. This was filtered and the filter cake washed with cold methanol (250 ml). Following drying at 50°C in a vacuum oven, 108.2 g (78%) of the title compound was obtained: IR (KBr) 1727, 1697, 1669 $cm^{-1}$, $^1H$ NMR (300 MHz, DMSO-$d_6$) $\delta$ 11.09 (s, NH), 7.65 (dd, aromatic H), 7.5 (d, aromatic H), 7.01 (d, aromatic H), 4.66 (s, ring $CH_2$), 3.87 (m, $CH_3C\underline{H}CH_2$), 3.54 (s, $CH_3O$), 2.77 (dd, one proton of $CH_3CHCH_2$), 2.48 (dd, one proton of $CH_3CHC\underline{H_2}$), 1.09 (d, $C\underline{H_3}CHCH_2$).

| | |
|---|---|
| Theor. $C_{14}H_{15}NO_5$: | C, 60.65; H, 5.45; N, 5.05 |
| Found: | C, 60.50; H, 5.49; N, 4.96 |

EXAMPLE 60

Resolution of 6-(3,4-Dihydro-3-oxo-l,4(2H)-benzoxazin-7-yl)-2,3,4,5-tetrahydro-5-methylpyridazin-3-one

The resolution of the titled compound was accomplished by chiral HPLC as outlined below.

The analytical separation was effected using the following equipment and method parameters.

| | |
|---|---|
| Column | ®-cyclodextran Astec cyclobond I 4.6 mm x 250 mm (serial #1742) |
| Mobile Phase | 30% methanol, 0.1% triethylamine, 0.05 N ammonium acetate adjusted to pH 6 with acetic acid. |
| Flow Rate | 0.7 ml/min at room temperature |
| Retention times | (+) isomer 11.4 min; (-) isomer 12.0 min |

The preparative separation was effected using the following equipment and method parameters.

| | |
|---|---|
| Column | ®-cyclodextran Astec cyclobond I 22.1 mm x 250 mm (serial #1924) |
| Mobile Phase | 30% methanol, 0.1% triethylamine, 0.05 N ammonium acetate adjusted to pH 6 with acetic acid. |
| Flow Rate | 6.0 ml/min at room temperature |

An average of 3.3 mg of the titled compound, dissolved in 0.2 ml of dimethylsulfoxide (DMSO), was injected onto the preparative column for each run. Seventeen runs were required to produce 25 mg each of (+) isomer and (-) isomer. Fractions containing the same enantiomer were pooled, evaporated, and run through a C-18 column to remove salts. The purity of the enantiomers was quantitated by measuring the area under the curve of the chiral analytical HPLC trace.

The dimethyl sulfoxide was removed under reduced pressure.

The purity was calculated by measuring the area under the HPLC curves.

(+) isomer: 95% pure (90% EE)

(-) isomer: 93% pure (86% EE)

The enantiomeric Excess (EE) (or the amount of the sample that is not racemic) was determined by subtracting the percentage of the lesser isomer from the percentage of the more abundant isomer.

95% - 5% = 90% EE, l0% racemate

93% - 7% = 86% EE, l4% racemate

Optical Rotation

Rotations were taken on a Rudolf Research Autopol III Automatic polarimeter in dimethylformamide (DMF) solution at 24°C and represent the rotation of plane polarized light at the sodium D line. Optical rotations were calculated using the formula: Optical rotation (α) = Spectrometer reading (R) x vol (ml) x l000/weight (mg).

| Fraction | A | B |
|---|---|---|
| isomer | (+) | (-) |
| Weight | 26.8 mg | 24.8 ml |
| Volume | 2.9 ml | 2.5 ml |
| Reading | +2.555 | -2.445 |
| [α] | +276.5° | -246.5° |

EXAMPLE 6l

Resolution of 6-(3,4-Dihydro-3-oxo-l,4(2H)-benzoxazin-7-yl)-2,3,4,5-tetrahydro-5-methylpyridazin-3-one

An additional method for resolving enantiomers of the titled compound, bemoradan, employed an HPLC method using a normal phase system.

HPLC CONDITIONS:

| | |
|---|---|
| Instrument: | Waters HPLC System |
| Column: | 25 cm x 4.6 mm Chiracel OD |
| Mobile Phase: | 80A:20B |
| | A=hexane |
| | B=isopropyl alcohol |
| Flow Rate: | 2 ml/min |
| Column temp: | Room temp. |
| Wavelength: | 320 nm; Detector sensitivity = 0.5 aufs |
| Sample solvent: | 2C:98D |
| | C=DMSO |
| | D=EtOAC |
| Retention times: | |
| | ll.9 min (-) Bemoradan |
| | l6.4 min (+) Bemoradan |

EXAMPLE 62

Resolution of methyl 4-(3,4-Dihydro-3-oxo-l,4(2H)-benzoxazin-7-yl)-3-methyl-4-oxobutyrate Precursor and the Conversion of Resolved Precursor to the (+) and (-) Enantiomers of Bemoradan

A. (±) Methyl 4-oxo-4-(3,4-dihydro-3-oxo-l,4(2H)-benzoxazin-7-yl)-3-methylbutyrate, which was prepared by the method of Example 59 was resolved by employing an HPLC method. The following HPLC conditions were employed using an analytical column.

HPLC CONDITIONS:

| | |
|---|---|
| Column: | 25 cm X 4.6 mm Chiracel OD |
| Mobile phase | : 90A:I0B |
| | A=hexane |
| | B=isopropyl alcohol |
| Flow: | I ml/min |
| Column temp: | Room temperature |
| Wavelength: | 320nm; Detector sensitivity = 0.5 aufs |
| Sample solvent: | thyl acetate |
| Sample Concn.: | 0 mg/ml |
| Sample Size: | 20 ul |
| Retention times: | I8.5 min (+) isomer |
| | 33.2 min (-) isomer |

Approximately I0-I5 mg of each enantiomeric sample of methyl 4-oxo-4-(3,4-dihydro-3-oxo-l,4(2H)-benzoxazin-7-yl)-3-methylbutyrate having ee purity = > 99% was collected and cyclized according to Example 8 to prepare the corresponding enantiomeric bemoradan compound. The enantiomeric purity of the (-) enantiomer of bemoradan so formed was analyzed using the same HPLC conditions as in Example 60. It was determined that enantiomeric purity went down from 97:3 for the (+) precursor to 90:I0 for the (-) bemoradan.

B. Methyl 4-oxo-4-(3,4-dihydro-3-oxo-l,4[2H]-benzoxazin-7-yl)-3-methylbutyrate was also resolved using a semi-preparative column.

HPLC CONDITIONS (semi preparative):

| | |
|---|---|
| Instrument: | Waters Delta 3000 LC |
| Column: | 50 cm X 5 cm Chiracel OD |
| Mobile phase | : 90:I0 Hexane:2-propanol |
| Flow: | 20 ml/min |
| Wavelength: | 320 nm |
| Sample solvent: | Ethylacetate |
| Sample conc..: | 30 mg/ml |
| Sample size: | 2 ml |
| Retention times: | 24.0 min (+) isomer |
| | 40.0 min (-) isomer |

Approximately 30-40 g of each enantiomer (ee purity = > 99%) was isolated and cyclized to the corresponding enantiomer of bemoradan. It was determined on analytical column that the cyclized samples lost a very small enantiomeric purity (< 0.5%) after cyclization. The physical preparation of the enantiomers of bemoradan along with the physical characterization of those enantiomers and the enantiomeric procursors follow.

C. Physical Data for the (-) Methyl 4-(3,4-dihydro-3-oxo-l,4(2H)-benzoxazin-7-yl)-3-methyl-3-oxobutyrate.

All physical data noted regarding the racemate in Example 59 was identical to that of the racemate except for the optical rotation: $[\alpha]^{22}$D-23° (l.0l, $CH_3OH$).

D. Physical Data for the (+) Methyl 4-(3,4-dihydro-3-oxo-l,4(2H)-benzoxazin-7-yl)-3-methyl-4-oxobuyrate

All physical data noted regarding the racemate in Example 59 was identical to that of the racemate except for the optical rotation: $(\alpha)^{22}$D+23°(l.0l, $CH_3OH$).

E. preparation of the (-) Bemoradan

A mixture of (+) methyl 4-(3,4-dihydro-3-oxo-l,4(2H)-benzoxazin-7-yl)-3-methyl-4-oxobutyrate (24.95 g, 0.09 mol), 55% hydrazine hydrate (20.98 g, 0.36 mol), ethanol (450 ml) and acetic acid (45 ml) was heated at 75-80°C for 3 hours. The initial slurry cleared upon reaching 50°C and then precipitated a solid which was present throughout the reaction period. The reaction was cooled to 25°C, filtered and the filter cake washed with ethanol (l00 ml). Drying yielded 22.2 g (95%) of crude material which was heated at reflux for 4 hours in ethanol (880 ml) as a slurry. This was filtered while hot and the resulting filter cake was dried in a vacuum oven at 50°C for 6 hours to yield l9.5 g (84%) of the title compound as an off white solid: $[\alpha]^{20}D$-423°(0.ll8, DMF); mp > 300°C; IR (KBr) l686, l620 $cm^{-l}$; MS (electron impact) m/z 259 (M+); $^{l}H$ NMR (300 MHz, DMSO-$d_6$) δ l0:9l and l0.88 (two s, NH), 7.39 (dd, aromatic H), 7.34 (d, aromatic H), 6.93 (d, aromatic H), 4.6l (s, $CH_2O$), 3.34 (m, $CH_3C\underline{H}CH_2$), 2.65 (dd, one proton of $CH_3CHC\underline{H_2}$) 2.22 (d, one proton of $CH_3CHC\underline{H_2}$), l.04 (d, $C\underline{H}_3CHCH_2$).

| | |
|---|---|
| Theor. $C_{l3}H_{l3}N_3O_3$: | C, 60.23; H, 5.05; N, l6.2l |
| Found: | C, 60.29; H, 4.96; N, l6.44 |

F. Preparation of the (+) Bemoradan

An identical reaction as in Example 62 (E.) above was carried out using (-) methyl 4-(3,4-dihydro-3-oxo-l,4(2H)-benzoxazin-7-yl)-3-methyl-4-oxobutyrate to yield l9.4 g (83%) of the title compound as an off white solid: $[\alpha]^{20}D$+420° (0.l08, DMF); all other physical data was identical to that obtained for the minus isomer.

EXAMPLE 63

Cardiotonic Activity

A. Racemate Testing Parameters

The cardiotonic activity of the compounds was determined in accordance with the method of Alousi, A. A., et al., J. Cir. Res., 45, 666 (l979). Basically, adult mongrel dogs were anesthetized with sodium pentobarbital and artifically respired. Arterial pressure was recorded via a femoral artery and the pressure pulse used to trigger a cardiotachometer for heart rate. Left ventricular pressure was measured with a Millar catheter and dP/dt was derived. Cardiac output was determined by measuring ascending aortic blood flow with an electromagnetic flow probe and myocardial contractile force was measured with a Walton Brodie strain gauge sutured to the right ventricle. Lead II EKG was also recorded. A standard dose of dopamine was administered to assess myocardial responsiveness. Test compounds were administered by i.v. infusion or bolus administration and the effects on cardiovascular parameters were determined. Dose-related effects of the test compound on BP, HR, dP/dt max., C.F. and C.O. were compared to pretreatment control values and expressed as a percentage change. The results are shown in Table II.

TABLE II

| Compound (Example) | Dose (mpk)[a] | CF[b] | $IC_{50}$[c] |
|---|---|---|---|
| 8 | 1.87 | 98 | 9.5 |
| 9 | 1.87 | 62 | 100 |
| 10 | 1.87 | 18 | 50 |
| 11 | 1.87 | 41 | N.T.[d] |
| 12 | 1.87 | 92 | 50 |
| 13 | 1.87 | 71 | 100 |
| 14 | 1.87 | 30 | 80 |
| 15 | 1.87 | 125 | 8 |
| 16 | 1.87 | 173 | 30 |
| 17 | 1.87 | 62 | 4 |
| 18 | 0.47 | 50 | 8 |
| 19 | 0.47 | 98 | 40 |
| 20 | 1.87 | 71 | 630 |
| 21 | 0.47 | 74 | 18 |
| 22 | 0.47 | 136 | 2 |
| 23 | 0.47 | 134 | 8 |
| 24 | 0.47 | 54 | 14 |
| 25 | 0.47 | 31 | 13 |
| 26 | 0.47 | 156 | 5 |
| 27 | 0.47 | 117 | 6 |
| 28 | 0.47 | 46 | 15 |
| 29 | 0.47 | 12 | 56 |
| 30 | 0.47 | 124 | 20 |
| 31 | 0.47 | 33 | 38 |
| 33 | 0.47 | 8 | 30 |
| 36 | 0.47 | 40 | 24 |
| 37 | 0.47 | 4 | 8 |
| 38 | 0.47 | 24 | 31 |
| 39 | 0.47 | 18 | 28 |
| 40 | 0.47 | 22 | 26 |
| 41 | 0.47 | 60 | 7 |
| 42 | 0.47 | 15 | 100 |
| 43 | 0.47 | 104 | 35 |
| 46 | 0.075 | 130 | 0.3 |
| 47 | 0.075 | 109 | 0.3 |
| 53 | 0.075 | 21 | 25 |
| 54 | 0.075 | 11 | 22 |
| 55 | 0.035 | 160 | <0.1 |
| 56 | 0.027 | 32 | <0.1 |

a I.V. dose used for cardiotonic activity assay.
b Percent increase in cardiac force.
c Micromolar concentration for 50% inhibition of cyclic nucleotide activity.
d Not tested.

B. Comparative Racemate and Enantiomer Testing Parameters

Adult mongrel dogs weighing approximately l0-l4 kg were anesthetized with sodium pentobarbital (35 mg/kg, i.p.) and artificially respired. A femoral artery was cannulated to measure mean arterial pressure (MAP) and a femoral vein was cannulated for administering compounds. A Millar microtip pressure transducer was placed in the left ventricle via the left carotid artery to measure left ventricular end diastolic pressure (EDP). pressure pulses triggered a cardiotachometer for determination of heart rate (HR) while the left ventricular pressure waveform was differentiated using a Gould differentiating amplifier to yield $dP/dt_{max}$, an index of contractility. A right thoracotomy was performed at the fourth intercostal space and the pericardial sac was opened. An externally calibrated Walton-Brodie strain gauge arch was sutured to the right ventricular free wall and stretched to produce a baseline force of approximately l00g. Subsequent changes in right ventricular contractile force (CF) were measured from this baseline. A Carolina electromagnetic flow probe was placed on the ascending aorta to estimate cardiac output (CO). A flexible fluid-filled catheter was inserted into the tip of the right atrium to measure right atrial pressure (RAP). Needle electrodes were placed in the appropriate limbs to record a lead II electrocardiogram (ECG). The following formulas were used to calculate derived parameters:

Stroke volume (SV) = CO/HR x l000
Stroke work (SW) = (RAP-EDP) x SV
Total peripheral resistance (TPR) = (MAP-EDP)/CO

Following surgery and stabilization of hemodynamic parameters, an i.v. infusion of dopamine (l0-l5 μg/kg/min for 3 min) was administered with a Harvard infusion pump to assess responsiveness to positive inotropic stimulation. Baseline hemodynamic values were obtained immediately prior to drug infusion. Responses were continuously monitored on a recorder (Gould 2800) and data collection was facilitated by the use of a Buxco cardiovascular analyzer and data logger.

In the comparative testing a stock solution of each enantiomer (l mg/ml) was prepared fresh daily and diluted before use. Compounds were dissolved in a small volume of dimethylformamide (DMF) and diluted with saline for a final concentration of l5% DMF. Further dilutions were made with a l5% DMF solution.

In the comparative testing each compound was administered in cumulative doses to separate dogs. Data were obtained at the end of each dose. (±) Bemoradan and (+) Bemordan were infused at cumulative doses of 5, l5, 35 and 75 μg/kg, administered with a Harvard infusion pump for 5 minutes each at rates of 0.56, l.l, 2.2, and l.l ml/min, respectively. (-) Bemoradan was given at doses of 5, l5 and 35 μg/kg following the same protocol. The high dose of the (-) isomer was not administered as a means of conserving compound.

Hemodynamic results were expressed as the percent change from pre-drug baseline values and compared using a Dunnett's test with a criterion for significance of $p < 0.05$. Contractile force $ED_{40}$ values (i.e., the dose which increased contractile force by 40%) were calculated from dose-response regression lines. The results are shown in Table III.

TABLE III

Dose-Dependent Hemodynamic Effects of Enantiomers of Bemoradan in Pentobarbital Anesthetized Open-Chest Dogs

(+) isomer

| | | Percent Change from Control | | | |
|---|---|---|---|---|---|
| Parameter | Baseline | 5 μg/kg | 15 μg/kg | 35 μg/kg | 75 μg/kg |
| MAP (mm Hg) | 123.2±7.4 | -1.4±1.1 | -1.8±1.7 | -2.2±2.2 | -3.8±2.8 |
| HR (bpm) | 133.0±9.1 | -0.7±0.8 | 1.2±0.8 | 4.5±1.0 | 10.6±3.7 |
| $dP/dt_{max}$ (mm Hg/sec) | 2049±187 | 2.8±1.0 | 7.3±1.8 | 18.9±3.6 | 37.0±6.4 |
| CF (g) | 95.2±2.6 | 4.3±2.7 | 11.8±3.3 | 29.5±5.2 | 59.5±7.2 |
| CO (l/min) | 1.6±0.2 | 2.0±1.4 | 4.8±2.8 | 10.6±5.9 | 13.4±8.5 |
| LVEDP (mm Hg) | 15.1±1.6 | -3.4±3.6 | -4.9±4.8 | -7.4±5.7 | -11.2±7.1 |
| RAP (mm Hg) | 2.9±0.7 | -10.6±8.5 | -5.8±9.7 | -4.2±8.4 | -21.7±13.3 |
| SV (ml) | 12.5±2.1 | 2.5±0.7 | 4.0±2.4 | 6.8±6.1 | 6.1±9.1 |
| SW (mm Hg©ml) | 1443±260 | 0.7±1.3 | 1.7±3.5 | 4.4±7.3 | 1.9±9.9 |
| TPR (mm Hg/l/min) | 73.7±9.6 | -3.6±0.7 | -6.6±1.7 | -11.3±3.7 | -14.4±5.3 |

- continued on next page -

Table III (cont.)

(-) isomer

| Parameter | Baseline | Percent Change from Control 5 μg/kg | 15 μg/kg | 35 μg/kg |
|---|---|---|---|---|
| MAP (mm Hg) | 122.4±8.0 | -7.4±3.4 | -12.8±4.5 | -18.7±4.3 |
| HR (bpm) | 136.4±8.2 | -3.1±0.5 | 10.5±1.2 | 17.1±3.6 |
| $dP/dt_{max}$ (mm Hg/sec) | 2743±412 | 11.7±5.6 | 41.6±12.3 | 58.0±14.5 |
| CF (g) | 95.8±4.2 | 28.3±4.4 | 119.9±18.8 | 187.2±28.9 |
| CO (l/min) | 1.8±0.1 | 2.9±0.5 | 0.8±1.9 | -3.4±2.4 |
| LVEDP (mm Hg) | 12.2±0.4 | -10.6±4.4 | -20.7±5.1 | -25.1±8.0 |
| RAP (mm Hg) | 3.9±1.0 | 3.8±11.4 | -0.9±16.4 | -1.4±16.0 |
| SV (ml) | 13.2±1.4 | 0.06±0.9 | -8.4±1.8 | -15.2±4.0 |
| SW (mm Hg©ml) | 1532±241 | -7.8±4.2 | -19.8±6.3 | -29.7±7.7 |
| TPR (mm Hg/l/min) | 63.1±4.2 | -10.2±5.2 | -12.8±7.5 | -14.9±5.1 |

Abbreviations

MAP = mean blood pressure
HR = heart rate
$dP/dt_{max}$ = peak rate of rise in left ventricular pressure over time
CF = contractile force
CO = cardiac output
LVEDP = left ventricular end diastolic pressure
RAP = right atrial pressure
SV = stroke volume
SW = stroke work
TRR = total peripheral resistance

The calculated $ED_{40}$'s (i.e., doses causing a 40% increase in contractility above baseline) for each of the isomers and the racemate is presented in Table IV. The (-) isomer was indistinguishable in potency from the racemate but almost an order of magnitude (7x) more potent than the (+) isomer.

TABLE IV

Inotropic Potency in vivo of Bemoradan and Its Enantiomers

| Bemoradan | CF $ED_{40}$ (µg/kg) with 95% F.L. |
|---|---|
| (-) | 5.7 (2.9 - 8.3) |
| (racemic) | 4.4 (3.9 - 4.9) |
| (+) | 42.3 (33.5 - 56.7) |

EXAMPLE 64

Phosphodiesterase Inhibitory Activity

A. Racemate Testing Parameter

The phosphodiesterase inhibitory activity was determined in accordance with the method of Thompson, W. J. et al., in Adv. Cycli. Nucleotide Res., Ed. Brooker, G. et al., Vol. I0, pp. 69-92 (I979). This assay measures the ability of compounds to inhibit cyclic nucleotide phosphodiesterase. This enzyme converts either cyclic AMP or cyclic GMP to the noncyclized AMP or GMP, respectively. Compounds were tested at various concentrations in the presence of cyclic AMP (0.I0-I.0 µM containing 0.2 µCi $^3$H-cyclic AMP), enzyme, and 0.05 M Tris-Cl buffer (pH 7.4, containing 5 mM $MgCl_2$). After a specified time, the reaction was stopped by heating to I00°C for one minute. After cooling, 0.I0 ml of a solution containing snake venom (I mg/ml) was added and the reaction was allowed to proceed for 30 minutes. Termination of this reaction was accomplished by the addition of I.0 ml of 33% Dowex slurry to separate the product from unconverted substrate. An aliquot was removed from the supernatant and quantitated by liquid scintillation spectrometry. The results are shown in Table II, supra, as the $IC_{50}$ which is the concentration (µM) of compound required to inhibit 50% of the cyclic nucleotide phosphodiesterase activity.

B. Comparative Racemate and Enantiomer Testing Parameters

Canine heart (I5.3 g) was homogenized for I min in I00 ml cold distilled, deionized water. After sonication for I min at 4°C, the crude material was centrifuged at 40,000 x g for 20 min. The supernatant was filtered through nylon mesh and applied to a DEAE cellulose column (2.5-25 cm) equilibrated with 70 mM sodium acetate (pH 6.5) buffer containing 5 mM ®-mercaptoethanol and 30% ethylene glycol. After applying the red, clear supernatant, two bed volumes of equilibration buffer were used to wash the column. Fractions I, II and III were eluted with 200 mM, 350 mM and 800 mM sodium acetate, respectively. All buffers contained the ®-mercaptoethanol and 30% ethylene glycol. The flow rate for the column was î60 ml/hr. Each fraction (tube) was I0 ml (2I0 drops). The fraction III isoenzyme was pooled and dialyzed against 2 liters of the equilibration buffer over a 6-7 hour period at 4°C. The material was aliquoted and stored at -20°C.

The cyclic AMP phosphodiesterase assay is essentially the one described by Thompson and Appleman (Biochemistry, I0, 3II (I97I). The enzyme, buffer (0.05 M Tris-Cl, pH 7.4 containing 5 mM $MgCl_2$) and compound were placed into plastic tubes (final volume 0.4 ml). Cyclic AMP was added (0.I µM) containing approximately 200,000 cpm $^3$H-cyclic AMP as a tracer. The enzyme reaction was allowed to proceed for 20 min at room temperature before being terminated by placing into a boiling water bath for 30 seconds. The tubes were removed and cooled. Snake venom (0.08 ml of a I mg/ml solution) was then added for 25 min. The snake venom contained a nucleotidase to convert all of the 5′ AMP (the product of the phosphodiesterase enzyme reaction) to uncharged adenosine. Resin was added to bind all of the excess cyclic AMP substrate. An aliquot (0.20 ml) was removed from the supernatant and placed into a scintillation vial containing 5 ml of scintillation cocktail. Radioactivity was determined in the scintillation counter, and the data were used to calculate enzyme activity.

The effects of (±), (+) and (-) bemoradan on inhibition of PDE III are shown in Table V. Both enantiomers were found to inhibit cardiac PDE III. The (-) isomer was approximately 4 times more potent than the racemate

and 44 times more potent than the (+) isomer.

TABLE V

Inhibition of PDE III by Racemic Bemoradan and Its Enantiomers

| Bemoradan | PDE III $IC_{50}$ (μM) |
|---|---|
| (-) | 0.18 |
| (racemate) | 0.71 |
| (+) | 8.0 |

EXAMPLE 65

Platelet Aggregation

The racemate and enantiomeric Bemoradan were tested for their effects in vitro on human platelet aggregation. Platelet rich plasma (PRP) was prepared from human donors who were free of aspirin for the preceding week. Venous blood was collected in sodium citrate vacutainer tubes and PRP was obtained by centrifuging the blood at l000 rpm for 5 min. Compounds were dissolved in methanol and added to 480 μl of PRP in a Chronolog dual channel aggregometer l min prior to aggregant. Serotonin at 5 μM or ADP at 2 μM were the aggregating agents.

The anti-aggregatory effect of the racemic Bemoradan and its enantiomers is shown in Table VI. The data show that the (-) isomer is l2-l6 times more potent than the (+) isomer while comparable in potency to the racemate as an inhibitor of agonist-induced platelet aggregation.

TABLE VI

Anti-aggregatory Potency of Bemoradan and Its Enantiomers

| Bemoradan | Antiaggregatory $IC_{50}$ (μM) vs. ADP | vs. 5-HT |
|---|---|---|
| (-) | 0.32 | 0.50 |
| (racemic) | 0.65 | 0.40 |
| (+) | 3.8 | 8.0 |

**Claims**

**1.** An enantiomer of a compound of the formula:

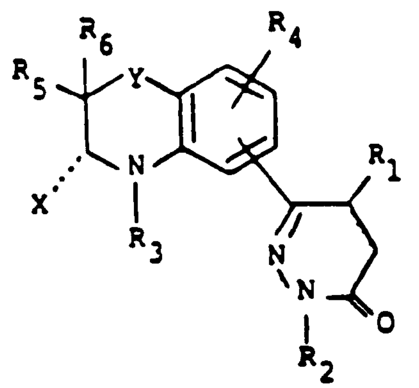

I

wherein

X is $H_2$ or O;

Y is O or S;

$R_l$ is $C_{l-6}$ straight- or branched-chain alkyl or $C_{3-6}$ cycloalkyl;

$R_2$ is H, $C_{l-6}$ straight- or branched-chain alkyl, $C_{3-6}$ cycloalkyl, or $C_{3-6}$ alkenyl;

$R_3$ is H, $C_{l-6}$ straight- or branched-chain alkyl or $C_{3-6}$ cycloalkyl, and when X is $H_2$, $R_3$ may also be $C_2$-$C_6$ acyl, arylacyl or alkanesulfonyl;

$R_4$ is H, halogen, $C_{l-6}$ straight- or branch-chain alkyl, $C_{3-6}$ cycloalkyl or $C_{l-6}$ alkoxy; and $R_5$ and $R_5$ are independently H, $C_{l-6}$ straight- or branched-chain alkyl or $C_{3-6}$ cycloalkyl.

**2.** The compound of claim I wherein the enantiomer has an enantiomeric orientation relative to the carbon bearing $R_l$.

**3.** The compound of claim 2 wherein the enantiomeric orientation is R.

**4.** The compound of claim 2 wherein the enantiomeric orientation is S.

**5.** The compound of claim I selected from the group consisting of 6-(3,4-dihydro-2-methyl-4-(l-methylethyl)-3-oxo-l,4(2H)-benzoxazin-6-yl)-2,3,4,5-tetrahydro-5-methylpyridazin-3-one; 6-(3,4-dihydro-4-cyclopentyl-2-methyl-3-oxo-l,4(2H)-benzoxazinyl-6-yl)-2,3,4,5-tetrahydro-5-methyl-pyridazin-3-one;6-(3,4-dihyd ro-6-methyl-3-oxo-l,4(2H)-benzoxazin-8-yl)-2,3,4,5-tetrahydro-5-methyl-pyridazin-3-one; 6-(3,4-dihydro-4,6-dimethyl-3-oxo-l,4(2H)-benzoxazin-8-yl)-2,3,4,5-tetrahydro-5-methylpyridan-3-one; and 6-(3,4-dihydro-6-methyl-l,4(2H)-benzoxazin-8-yl)-2,3,4,5-tetrahydro-5-methylpyridazin-3-one.

**6.** The compound of claim I selected from the group consisting of 6-(3,4-dihydro-4,7-dimethyl-3-oxo-l,4(2H)-benzoxazin-6-yl)-2,3,4,5-tetrahydro-5-methylpyridazin-3-one; 6-(3,4-dihydro-2,7-dimethyl-3-oxo-l,4(2H)-benzoxazin-6-yl)-2,3,4,5-tetrahydro-5-methylpyridazin-3-one; and 6-(3,4-dihydro-2,4,7-trimethyl-3-oxo-l,4(2H)-benzoxazin-6-yl)-2,3,4,5-tetrahydro-5-methylpyridazin-3-one.

**7.** The compound of claim I selected from the group consisting of 6-(3,4-dihydro-l,4(2H)-benzoxazin-6-yl)-2,3,4,5-tetrahydro-5-methylpyridazin-3-one; 6-(4-acetyl-3,4-dihydro-l,4(2H)-benzoxazin-6-yl)-2,3,4,5-tetrahydro-5-methylpyridazin-3-one; 6-(3,4-dihydro-4-methanesulfonyl-l,4(2H)-benzoxazin-6-yl)-2,3,4,5-tetrahydro-5-methylpyridazin-3-one; 6-(3,4-dihydro-2-methyl-l,4(2H)-benzoxazin-6-yl)-2,3,4,5-tetrahydro-5-methylpyridazin-3-one; 6-(3,4-dihydro-3-oxo-l,4(2H)-benzoxazin-6-yl)-2,3,4,5-tetrahydro-5-methylpyradazin-3-one; 6-(3,4-dihydro-4-methyl-3-oxo-l,4 (2H)-benzoxazin-6-yl)-2,3,4,5-tetrahydro-5-methylpyridazin-3-one; 6-(3,4-dihydro-2,4-dimethyl-3-oxo-l,4(2H)-benzoxazin-6-yl)-2,3,4,5-tetrahydro-5-methylpyridazin-3-one; 6-(3,4-dihydro-7-methyl-3-oxo-l,4(2H)-benzoxazin-6-yl)-2,3,4,5-tetrahydro-5-methylpyridazin-3-one; and 6-(3,4-dihydro-2-methyl-3-oxo-l,4(2H)-benzoxazin-6-yl)-2,3,4,5-tetrahydro-5-methylpyridazin-3-one.

**8.** The compound of claim I selected from the group consisting of 6-(3,4-dihydro-3-oxo-l,4(2H)-benzoxazin-7-yl)-2,3,4,5-tetrahydro-5-methylpyridazin-3-one; 6-(3,4-dihydrod--methyl-3-oxo-l,4(2H)-benzoxazin-7-yl)-2,3,4,5-tetrahydro-5-methylpyridazin-3-one; 6-(3,4-dihydro-2-methyl-3-oxo-l,4(2H)-benzoxazin-7-yl)-2,3,4,5-tetrahydro-5-methylpyridazin-3-one; 6-(3,4-dihydro-2,4-dimethyl-3-oxo-l,4(2H)-benzoxazin-7-yl)-2,3,4,5-tetrahydro-5-methylpyridazin-3-one; 6-(3,4-dihydro-2,2-dimethyl-3-oxo-l,4(2H)-benzoxazin-7-yl)-2,3,4,5-tetrahydro-5-methylpyridazin-3-one; 6-(3,4-dihydro-2,2,4-trimethyl-3-oxo-l,4(2H)-benzoxazin-7-yl)-2,3,4,5-tetrahydro-5-methylpyridazin-3-one; 6-(3,4-dihydro-3-oxo-l,4[2H]-benzoxazin-7-yl)-2,3,4,5-tetrahydro-5-methyl-pyridazin-3-one; and 6-(3,4-dihydro-4-methyl-3-oxo-l,4[2H]-benzoxazin-7-yl-2,3,4,5-tetrahydro-5-methyl-pyridazin-3-one.

**9.** The compound of claim I which is 6-(3,4-dihydro-4-methyl-3-oxo-l,4[2H]-benzoxazin-7-yl)-2,3,4,5-tetrahydro-5-methylpyridazin-3-one.

**10.** The compound of claim 8 wherein the enantiomer has an enantiomeric orientation relative to the carbon bearing $R_l$ that is R.

**Claims for the following Contracting States : ES GR**

**1.** A process for the production of a desired enantiomer of a compound of the formula:

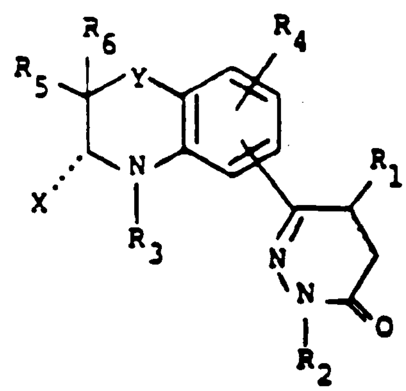

wherein

X is $H_2$ or O;

Y is O or S;

$R_l$ is $C_{l-6}$ straight- or branched-chain alkyl or $C_{3-6}$ cycloalkyl;

$R_2$ is H, $C_{l-6}$ straight- or branched-chain alkyl, $C_{3-6}$ cycloalkyl, or $C_{3-6}$ alkenyl;

$R_3$ is H, $C_{l-6}$ straight- or branched-chain alkyl or $C_{3-6}$ cycloalkyl, and when X is $H_2$, $R_3$ may also be $C_2$-$C_6$ acyl, arylacyl or alkanesulfonyl;

$R_4$ is H, halogen, $C_{l-6}$ straight- or branch-chain alkyl, $C_{3-6}$ cycloalkyl or $C_{l-6}$ alkoxy; and $R_5$ and $R_5$ are independently H, $C_{l-6}$ straight- or branched-chain alkyl or $C_{3-6}$ cycloalkyl,

which process comprises the steps of:

(a) preparing a mixture of enantiomers of the compound or of an optically active precursor thereof, and

(b) resolving the mixture to obtain the desired enantiomer of the compound or of the precursor thereof substantially free from other enantiomers.

**2.** The process of claim I wherein the enantiomer has an enantiomeric orientation relative to the carbon bearing $R_l$.

**3.** The process of claim 2 wherein the enantiomeric orientation of the desired enantiomer is R.

**4.** The process of claim 2 wherein the enantiomeric orientation of the desired enantiomer is S.

**5.** The process of claim I wherein the compound is selected from the group consisting of 6-(3,4-dihydro-2-methyl-4-(l-methylethyl)-3-oxo-l,4(2H)-benzoxazin-6-yl)-2,3,4,5-tetrahydro-5-methylpyridazin-3-one; 6-(3,4-dihydro-4-cyclopentyl-2-methyl-3-oxo-l,4(2H)-benzoxazinyl-6-yl)-2,3,4,5-tetrahydro-5-methyl-pyridazin-3-one; 6-(3,4-dihydro-6-methyl-3-oxo-l,4(2H)-benzoxazin-8-yl)-2,3,4,5-tetrahydro-5-methyl-pyridazin-3-one; 6-(3,4-dihydro-4,6-dimethyl-3-oxo-l,4(2H)-benzoxazin-8-yl)-2,3,4,5-tetrahydro-5-methylpyridan-3-one; and 6-(3,4-dihydro-6-methyl-l,4(2H)-benzoxazin-8-yl)-2,3,4,5-tetrahydro-5-methylpyridazin-3-one.

6. The process of claim I wherein the compound is selected from the group consisting of 6-(3,4-dihydro-4,7-dimethyl-3-oxo-l,4(2H)-benzoxazin-6-yl)-2,3,4,5-tetrahydro-5-methylpyridazin-3-one; 6-(3,4-dihydro-2,7-dimethyl-3-oxo-l,4(2H)-benzoxazin-6-yl)-2,3,4,5-tetrahydro-5-methylpyridazin-3-one; and 6-(3,4-dihydro-2,4,7-trimethyl-3-oxo-l,4(2H)-benzoxazin-6-yl)-2,3,4,5-tetrahydro-5-methylpyridazin-3-one.

7. The process of claim I wherein the compound is selected from the group consisting of 6-(3,4-dihydro-l,4(2H)-benzoxazin-6-yl)-2,3,4,5-tetrahydro-5-methylpyridazin-3-one; 6-(4-acetyl-3,4-dihydro-l,4(2H)-benzoxazin-6-yl)-2,3,4,5-tetrahydro-5-methylpyridazin-3-one; 6-(3,4-dihydro-4-methanesulfonyl-l,4(2H)-benzoxazin-6-yl)-2,3,4,5-tetrahydro-5-methylpyridazin-3-one; 6-(3,4-dihydro-2-methyl-l,4(2H)-benzoxazin-6-yl)-2,3,4,5-tetrahydro-5-methylpyridazin-3-one; 6-(3,4-dihydro-3-oxo-l,4(2H)-benzoxazin-6-yl)-2,3,4,5-tetrahydro-5-methyl-pyradazin-3-one; 6-(3,4-dihydro-4-methyl-3-oxo-l,4(2H)-benzoxazin-6-yl)-2,3,4,5-tetrahydro-5-methylpyridazin-3-one; 6-(3,4-dihydro-2,4-dimethyl-3-oxo-l,4(2H)-benzoxazin-6-yl)-2,3,4,5-tetrahydro-5-methylpyridazin-3-one; 6-(3,4-dihydro-7-methyl-3-oxo-l,4(2H)-benzoxazin-6-yl)-2,3,4,5-tetrahydro-5-methylpyridazin-3-one; and 6-(3,4-dihydro-2-methyl-3-oxo-l,4(2H)-benzoxazin-6-yl)-2,3,4,5-tetrahydro-5-methylpyridazin-3-one.

8. The process of claim I wherein the compound is selected from the group consisting of 6-(3,4-dihydro-3-oxo-l,4(2H)-benzoxazin-7-yl)-2,3,4,5-tetrahydro-5-methylpyridazin-3-one; 6-(3,4-dihydro-4-methyl-3-oxo-l,4(2H)-benzoxazin-7-yl)-2,3,4,5-tetrahydro-5-methylpyridazin-3-one; 6-(3,4-dihydro-2-methyl-3-oxo-l,4(2H)-benzoxazin-7-yl)-2,3,4,5-tetrahydro-5-methylpyridazin-3-one; 6-(3,4-dihydro-2,4-dimethyl-3-oxo-l,4(2H)-benzoxazin-7-yl)-2,3,4,5-tetrahydro-5-methylpyridazin-3-one; 6-(3,4-dihydro-2,2-dimethyl-3-oxo-l,4(2H)-benzoxazin-7-yl)-2,3,4,5-tetrahydro-5-methylpyridazin-3-one; 6-(3,4-dihydro-2,2,4-trimethyl-3-oxo-l,4(2H)-benzoxazin-7-yl)-2,3,4,5-tetrahydro-5-methylpyridazin-3-one; 6-(3,4-dihydro-3-oxo-l,4[2H]-benzoxazin-7-yl)-2,3,4,5-tetrahydro-5-methyl-pyridazin-3-one; and 6-(3,4-dihydro-4-methyl-3-oxo-l,4[2H]-benzoxazin-7-yl-2,3,4,5-tetrahydro-5-methyl-pyridazin-3-one.

9. The process of claim I wherein the compound is 6-(3,4-dihydro-4-methyl-3-oxo-l,4[2H]-benzoxazin-7-yl)-2,3,4,5-tetrahydro-5-methylpyridazin-3-one.

10. The process of claim 8 wherein the desired enantiomer has an enantiomeric orientation relative to the carbon bearing $R_l$ that is R.

European Patent Office

# EUROPEAN SEARCH REPORT

Application Number

EP 92 30 3505

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | EP-A-0 272 914 (ORTHO PHARMACEUTICAL)<br>* page 13 - page 25 *<br>--- | 1,2,5-9 | C07D413/04<br>C07D417/04<br>A61K31/50 |
| X | JOURNAL OF MEDICINAL CHEMISTRY.<br>vol. 33, no. 1, 1990, WASHINGTON US<br>pages 380 - 386;<br>D. COMBS ET AL.:<br>'6-BENZOXAZINYLPYRIDAZIN-3-ONES: POTENT, LONG-ACTING POSITIVE INOTROPE AND PERIFERAL VASODILATATOR AGENT'<br>* the whole document *<br>----- | 1,2,5-9 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 19 JUNE 1992 | FRANCOIS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)